(19) Europäisches Patentamt / European Patent Office / Office européen des brevets

(11) **EP 1 558 569 B1**

(12) **EUROPÄISCHE PATENTSCHRIFT**

(45) Veröffentlichungstag und Bekanntmachung des Hinweises auf die Patenterteilung:
**30.09.2009 Patentblatt 2009/40**

(51) Int Cl.:
***C07C 253/24*** *(2006.01)*    ***B01J 23/00*** *(2006.01)*
***B01J 23/20*** *(2006.01)*    ***C07C 51/215*** *(2006.01)*

(21) Anmeldenummer: **03808715.1**

(22) Anmeldetag: **09.10.2003**

(86) Internationale Anmeldenummer:
**PCT/EP2003/011144**

(87) Internationale Veröffentlichungsnummer:
**WO 2004/035527 (29.04.2004 Gazette 2004/18)**

(54) **MULTIMETALLOXIDMASSEN**

MULTIMETALLIC OXIDE COMPOSITION

MASSES D'OXYDE MULTIMETALLIQUE

(84) Benannte Vertragsstaaten:
**AT BE BG CH CY CZ DE DK EE ES FI FR GB GR HU IE IT LI LU MC NL PT RO SE SI SK TR**

(30) Priorität: **17.10.2002 DE 10248584**
        **20.11.2002 DE 10254278**
        **20.11.2002 DE 10254279**

(43) Veröffentlichungstag der Anmeldung:
**03.08.2005 Patentblatt 2005/31**

(73) Patentinhaber: **BASF SE**
**67056 Ludwigshafen (DE)**

(72) Erfinder:
 • **BORGMEIER, Frieder**
**68163 Mannheim (DE)**
 • **DIETERLE, Martin**
**68167 Mannheim (DE)**
 • **HIBST, Hartmut**
**69198 Schriesheim (DE)**

(56) Entgegenhaltungen:
EP-A- 1 192 987        EP-A- 1 254 707
EP-A- 1 254 709        EP-A- 1 254 710
WO-A-02/06199        WO-A-02/051539

 • **WATANABE H ET AL: "New synthesis route for Mo-V-Nb-Te mixed oxides catalyst for propane ammoxidation" APPLIED CATALYSIS A: GENERAL, ELSEVIER SCIENCE, AMSTERDAM, NL, Bd. 194-195, 13. März 2000 (2000-03-13), Seiten 479-485, XP004272252 ISSN: 0926-860X**

**Beschreibung**

Vorliegende Erfindung betrifft Multimetalloxidmassen der allgemeinen Stöchiometrie I

$$MO_1V_aM^1_bM^2_cM^3_dO_n \qquad (I),$$

mit

M$^1$ = wenigstens eines der Elemente aus der Gruppe umfassend Te und Sb;
M$^2$ = wenigstens eines der Elemente aus der Gruppe umfassend Nb, Ti, W, Ta und Ce;
M$^3$ = wenigstens eines der Elemente aus der Gruppe umfassend Pb, Ni, Co, Bi, Pd, Ag, Pt, Cu, Au, Ga, Zn, Sn, In, Re, Ir, Sm, Sc, Y, Pr, Nd und Tb;
a = 0,01 bis 1,
b = > 0 bis 1,
c = > 0 bis 1,
d = > 0 bis 0,5 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2Θ 22,2 $\pm$ 0,5° (h), 27,3 $\pm$ 0,5° (i) und 28,2 $\pm$ 0,5° (k) liegen, wobei

- der Beugungsreflex h innerhalb des Röntgendiffraktogramms der intensitätsstärkste ist, sowie eine Halbwertsbreite von höchstens 0,5° aufweist,
- die Intensität $P_i$ des Beugungsreflexes i und die Intensität $P_k$ des Beugungsreflexes k die Beziehung 0,65 $\leq$ R $\leq$ 0,85 erfüllen, in der R das durch die Formel

$$R = P_i \; / \; (P_i + P_k)$$

definierte Intensitätsverhältnis ist, und
- die Halbwertsbreite des Beugungsreflexes i und des Beugungsreflexes k jeweils $\leq$ 1° beträgt und die dadurch gekennzeichnet sind, daß

die wenigstens eine Multimetalloxidmasse (I) eine solche ist, deren Röntgendiffraktogramm keinen Beugungsreflex mit der Scheitelpunktlage 2Θ = 50,0 $\pm$ 0,3° aufweist.

Ferner betrifft vorliegende Erfindung die Herstellung von Multimetalloxidmassen (I) und deren Verwendung zur heterogen katalysierten partiellen Oxidation und/oder Ammoxidation von gesättigten und/oder ungesättigten Kohlenwasserstoffen.

Multimetalloxidmassen der allgemeinen Stöchiometrie (I) und mit einem stöchiometrischen Koeffizienten d = 0, die dadurch erhältlich sind, dass man ein inniges Trockengemisch ihrer elementaren Konstituenten in einer Sauerstoff enthaltenden Atmosphäre calciniert, sind z.B. aus der EP-A 318295 bekannt. Sie eignen sich z.B. für die heterogen katalysierte Ammoxidation von Propan oder iso-Butan zur Herstellung von Acrylnitril bzw. Methacrylnitril und sind dadurch gekennzeichnet, daß sie einen hohen amorphen Strukturanteil aufweisen.

Aus der EP-A 512846 ist bekannt, dass die Performance der Multimetalloxidmassen der EP-A 318295 durch Zusatz von Promotorelementen M$^3$ im Hinblick auf ihre Verwendung als Katalysatoren für die partielle Ammoxidation von gesättigten Kohlenwasserstoffen verbessert werden kann.

Aus der EP-A 529853, der EP-A 603836, der EP-A 608838, der EP-A 767164, der EP-A 895809 und der EP-A 962253 sind Multimetalloxidmassen der allgemeinen Stöchiometrie (I) und mit einem stöchiometrischen Koeffizienten d = 0 bekannt, die dadurch erhältlich sind, dass man ein inniges Trockengemisch ihrer elementaren Konstituenten in einer an Sauerstoff im wesentlichen freien Atmosphäre calciniert. Sie eignen sich im Vergleich mit den Multimetalloxidmassen der EP-A 318295 und der EP-A 512846 noch besser als Katalysatoren für die heterogen katalysierte partielle Ammoxidation und/oder Oxidation von gesättigten Kohlenwasserstoffen. Letzteres insbesondere dann, wenn das innige Trockengemisch als Katalysatorvorläufer durch Sprühtrocknung erzeugt wurde.

Dies wird in den zitierten Schriften darauf zurückgeführt, dass diese Multimetalloxidmassen herstellungsbedingt weitgehend in einer spezifischen Kristallstruktur kristallin vorliegen, die dadurch gekennzeichnet ist, daß ihr Röntgendiffraktogramm bei den 2θ-Scheitelpunkt-Lagen 22,1 $\pm$ 0,3°, 28,2 $\pm$ 0,3°, 36,2 $\pm$ 0,3°, 45,2 $\pm$ 0,3° und 50,0 $\pm$ 0,3°

intensitätsstarke Beugungsreflexe aufweist.

Aus der Lehre der DE-A 19835247, der EP-A 1090684 und der WO 0206199 ist bekannt, dass die vorgenannte spezifische Kristallstruktur lediglich eine kristalline Phase bildet, in welcher solche Multimetalloxidmassen auftreten können. Diese kristalline Phase wird in der vorgenannten Literatur in der Regel als k-Phase bezeichnet.

Eine zweite spezifische Kristallstruktur in der die relevanten Multimetalloxidmassen auftreten können wird in der Regel als i-Phase bezeichnet. Ihr Röntgendiffraktogramm ist gemäß den vorgenannten Schriften u.a. dadurch gekennzeichnet, dass es bei den $2\Theta$-Scheitelpunkt-Lagen 22,2 $\pm$ 0,4°, 27,3 $\pm$ 0,4° und 28,2 $\pm$ 0,4° intensitätsstarke Beugungsreflexe aufweist, im Unterschied zur k-Phase bei der $2\Theta$-Scheitelpunkt-Lage 50,0 $\pm$ 0,3° jedoch keinen Beugungsreflex zeigt.

Zum Beispiel gemäß der Lehre der EP-A 529853, der EP-A 608838 sowie der EP-A 603836 zeichnet die k-Phase für die katalytische Aktivität der dort genannten Multimetalloxidmassen verantwortlich.

Bei der wie oben beschriebenen Herstellweise werden nun normalerweise weder reine k-Phase noch reine i-Phase sondern Mischkristallstrukturen erhalten, die ein verwachsenes Gemisch aus k- und i-Phase sind.

In der EP-A 1192987, der EP-A 1192986, der EP-A 1192983 und der EP-A 1192982 werden solche Mischkristall-Multimetalloxidmassen hergestellt und gezeigt, daß ihre Performance durch Zusatz von Promotorelementen $M^3$ im Hinblick auf ihre Verwendung als Katalysatoren für die partielle Ammoxidation und/oder Oxidation von gesättigten Kohlenwasserstoffen verbessert werden kann, wobei der k-Phase die entscheidende Rolle zugemessen wird.

Im Unterschied dazu mißt die JP-A 11-169716 sowohl der k-Phase als auch der i-Phase eine entscheidende Rolle für die katalytische Aktivität solcher Mischkristall-Multimetalloxidmassen bei der partiellen Ammoxidation von gesättigten Kohlenwasserstoffen bei. Danach ist die k-Phase für eine befriedigende Selektivität der Nitrilbildung und die i-Phase für einen ausreichenden Umsatz des gesättigten Kohlenwasserstoff verantwortlich.

In "Ammoxidation of propane over Mo-V-Nb-Te mixed oxide catalysts" aus "Spillover and Migration of Surface on Catalysts" Ed. by Can Li und Quin Xin, Elsevier Science B.V. (1997), S. 473 ff untermauern die Erfinder der JP-A 11-169716 diese Vorstellung, die auch durch die Lehre der DE-A 19835247 und der EP-A 895089 gestützt wird.

Aus der JP-A 7-232071 und der WO 0206199 ist dem gegenüber bekannt, dass auch ausschließlich in der i-Phase-Struktur vorliegende Multimetalloxidmassen als Katalysatoren für die heterogen katalysierte partielle Ammoxidation und/oder Oxidation von gesättigten Kohlenwasserstoffen geeignet sind.

Es wurden ferner schon Versuche durchgeführt, in denen gezeigt wird, daß ausschließlich in der k-Phase Struktur vorliegende Multimetalloxidmassen katalytisch inaktiv sind und die Vorstellung der JP-A 11-169716 stützen, wonach die i-Phase für die Aktivität und die k-Phase lediglich für eine Maximierung der Selektivität verantwortlich zeichnet.

Aus der WO 00/29106, der WO 00/29105, der WO 00/38833 und der WO 00/69802 sind Promotoren enthaltende Multimetalloxidmassen bekannt, die eine im wesentlichen amorphe Struktur aufweisen, die im Röntgendiffraktogramm in Form von sehr breiten Beugungsreflexen abgebildet wird, und die ebenfalls als Katalysatoren für Partialoxidationen empfohlen werden.

Aus der DE-A 10118814 bzw. PCT/EP/02/04073 ist bekannt, daß reine i-Phase Mulitmetalloxidmassen auch geeignete Katalysatoren für partielle Oxidationen von ungesättigten Kohlenwasserstoffen sind.

Aus der JP-A 8-57319 ist bekannt, daß Mo und/oder V enthaltende Multimetalloxidaktivmassen durch Behandlung mit Säure aktiviert werden können.

Nachteilig an den gegebenen Lehren des Standes der Technik ist jedoch, daß sie einerseits die Frage offen lassen, ob die Promotoren sowohl in die i- als auch in die k-Phase eingebaut werden und ob sie die katalytische Wirksamkeit beider Phasen beeiflussen und andererseits ihre Multimetalloxidmassen bezüglich der Selektivität der Zielproduktbildung als Katalysatoren für die heterogen katalysierte partielle Oxidation und/oder Ammoxidation von gesättigten und/oder ungesättigten Kohlenwasserstoffen nicht im vollen Umfang zu befriedigen vermögen.

Die Aufgabe der vorliegenden Erfindung bestand daher darin, die offene Frage zu beantworten und im Sinne der Aufgabe verbesserte Multimetalloxidmassen zur Verfügung zu stellen.

Demgemäß wurden die eingangs definierten Multimetalloxidmassen (I) gefunden (alle in dieser Schrift auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall ($2\Theta$):0,02°, Meßzeit je Schritt: 2,4 s, Detektor: Scintillationszählrohr); die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition; das gleiche gilt für die Definition der Halbwertsbreite).

Erfindungsgemäß bevorzugt gilt $0,67 \leq R \leq 0,75$ und ganz besonders bevorzugt gilt R = 0,69 bis 0,75 bzw. R = 0,71 bis 0,74, bzw. R = 0,72.

Neben den Beugungsreflexen h, i und k enthält das Röntgendiffraktogramm von erfindungsgemäßen Multimetalloxidmassen (I) in der Regel noch weitere Beugungsreflexe, deren Scheitelpunkte bei den nachfolgenden Beugungswinkeln ($2\Theta$) liegen:

9,0 ± 0,4° (l)
6,7 ± 0,4° (o) und
7,9 ± 0,4° (p).

Günstig ist es ferner, wenn das Röntgendiffraktogramm zusätzlich einen Beugungsreflex enthält, dessen Scheitelpunkt beim Beugungswinkel (2Θ) = 45,2 ± 0,4° (q) liegt.

Häufig enthält das Röntgendiffraktogramm von Multimetalloxidmassen (I) auch noch die Reflexe 29,2 ± 0,4° (m) und 35,4 ± 0,4° (n) (Scheitelpunktlagen).

Ordnet man dem Beugungsreflex h die Intensität 100 zu, ist es erfindungsgemäß günstig, wenn die Beugungsreflexe i, l, m, n, o, p, q in der gleichen Intensitätsskala die nachfolgenden Intensitäten aufweisen:

i: 5 bis 95, häufig 5 bis 80, teilweise 10 bis 60;
l: 1 bis 30;
m: 1 bis 40;
n: 1 bis 40;
o: 1 bis 30;
p: 1 bis 30 und
q: 5 bis 60.

Enthält das Röntgendiffraktogramm der erfindungsgemäßen Multimetalloxidmassen (I) von den vorgenannten zusätzlichen Beugungsreflexen, ist die Halbwertsbreite derselben in der Regel ≤ 1°.

Die spezifische Oberfläche von erfindungsgemäßen Multimetalloxidmassen (I) beträgt vielfach 1 bis 40 $m^2/g$, oft 11 bzw. 12 bis 40 $m^2/g$ und häufig 15 bzw. 20 bis 40 bzw. 30 $m^2/g$ (bestimmt nach der BET-Methode, Stickstoff).

Erfindungsgemäß bevorzugt beträgt der stöchiometrische Koeffizient a der erfindungsgemäßen Multimetalloxidmassen (I), unabhängig von den Vorzugsbereichen für die anderen stöchiometrischen Koeffizienten der Multimetalloxidmassen (I), 0,05 bis 0,6, besonders bevorzugt 0,1 bis 0,6 oder 0,5.

Unabhängig von den Vorzugsbereichen für die anderen stöchiometrischen Koeffizienten der Multimetalloxidmassen (I) beträgt der stöchiometrische Koeffizient b bevorzugt 0,01 bis 1, und besonders bevorzugt 0,01 bzw. 0,1 bis 0,5 oder 0,4.

Der stöchiometrische Koeffizient c der erfindungsgemäßen Multimetalloxidmassen (I) beträgt, unabhängig von den Vorzugsbereichen für die anderen stöchiometrischen Koeffizienten der Multimetalloxidmassen (I) 0,01 bis 1 und besonders bevorzugt 0,01 bzw. 0,1 bis 0,5 oder 0,4. Ein ganz besonders bevorzugter Bereich für den stöchiometrischen Koeffizienten c, der, unabhängig von den Vorzugsbereichen für die anderen stöchiometrischen Koeffizienten der erfindungsgemäßen Multimetalloxidmassen (I), mit allen anderen Vorzugsbereichen in dieser Schrift kombinierbar ist, ist der Bereich 0,05 bis 0,2.

Erfindungsgemäß bevorzugt beträgt der stöchiometrische Koeffizient d der erfindungsgemäßen Multimetalloxidmassen (I), unabhängig von den Vorzugsbereichen für die anderen stöchiometrischen Koeffizienten der Multimetalloxidmassen (I), 0,00005 bzw. 0,0005 bis 0,5, besonders bevorzugt 0,001 bis 0,5, häufig 0,002 bis 0,3 und oft 0,005 bzw. 0,01 bis 0,1.

Besonders günstig sind erfindungsgemäße Multimetalloxidmassen (I), deren stöchiometrische Koeffizienten a, b, c und d gleichzeitig im nachfolgenden Raster liegen:

a = 0,05 bis 0,6;
b = 0,01 bis 1 (bzw. 0,01 bis 0,5);
c = 0,01 bis 1 (bzw. 0,01 bis 0,5); und
d = 0,0005 bis 0,5 (bzw. 0,001 bis 0,3).

Ganz besonders günstig sind erfindungsgemäße Multimetalloxidmassen (I), deren stöchiometrische Koeffizienten a, b, c und d gleichzeitig im nachfolgenden Raster liegen:

a = 0,1 bis 0,6;
b = 0,1 bis 0,5;
c = 0,1 bis 0,5; und
d = 0,001 bis 0,5, bzw. 0,002 bis 0,3, bzw. 0,005 bis 0,1.

$M^1$ ist bevorzugt Te.

Alles vorgenannte gilt vor allem dann, wenn $M^2$ wenigstens zu 50 mol-% seiner Gesamtmenge Nb und ganz besonders bevorzugt dann, wenn $M^2$ zu wenigstens 75 mol-% seiner Gesamtmenge, bzw. zu 100 mol-% seiner Gesamtmenge Nb ist.

Es gilt vor allem aber auch, unabhängig von der Bedeutung von $M^2$, dann, wenn $M^3$ wenigstens ein Element aus der Gruppe umfassend Ni, Co, Bi, Pd, Ag, Au, Pb und Ga oder wenigstens ein Element aus der Gruppe umfassend Ni, Co, Pd und Bi ist.

Alles vorgenannte gilt vor allem aber auch dann, wenn $M^2$ wenigstens zu 50 mol-% seiner Gesamtmenge, oder zu wenigstens 75 mol-%, oder zu 100 mol-% Nb und $M^3$ wenigstens ein Element aus der Gruppe umfassend Ni, Co, Bi, Pd, Ag, Au, Pb und Ga ist.

Alles vorgenannte gilt vor allem aber auch dann, wenn $M^2$ wenigstens zu 50 mol-%, oder zu wenigstens 75 mol-%, oder zu 100 mol-% seiner Gesamtmenge Nb und $M^3$ wenigstens ein Element aus der Gruppe umfassend Ni, Co, Pd und Bi ist.

Ganz besonders bevorzugt gelten alle Aussagen hinsichtlich der stöchiometrischen Koeffizienten dann, wenn $M^1$ = Te, $M^2$ = Nb und $M^3$ = wenigstens ein Element aus der Gruppe umfassend Ni, Co und Pd.

Weitere erfindungsgemäß geeignete Stöchiometrien sind jene, die für die Multimetalloxidmassen der Stöchiometrie (I) im eingangs zitierten Stand der Technik offenbart sind.

Das Prinzip eines gezieltes Verfahrens zur Herstellung von erfindungsgemäßen Multimetalloxidmassen (I) offenbart z.B. die WO 0206199 und die in dieser Schrift zitierten Literaturzitate. Danach wird in an sich bekannter Weise zunächst eine Multimetalloxidmasse erzeugt, die die Stöchiometrie (I) aufweist, aber ein in der Regel innig verwachsenes Mischkristallsystem aus i-Phase und anderen Phasen (z.B. k-Phase) ist. Aus diesem Gemisch kann nun der Anteil an i-Phase dadurch isoliert werden, daß man die anderen Phasen, z.B. die k-Phase, mit geeigneten Flüssigkeiten herauswäscht. Als solche Flüssigkeiten kommen z.B. wäßrige Lösungen organischer Säuren (z.B. Oxalsäure, Ameisensäure, Essigsäure, Zitronensäure und Weinsäure), anorganische Säuren (z.B. Salpetersäure), Alkohole und wäßrige Wasserstoffperoxidlösungen in Betracht. Desweiteren offenbart auch die JP-A 7-232071 ein Verfahren zur Herstellung von i-Phase-Multimetalloxidmassen.

Mischkristallsysteme aus i- und k-Phase werden in der Regel nach den im Stand der Technik beschriebenen Herstellverfahren erhalten (vgl. z.B. DE-A 19835247, EP-A 529853, EP-A 603836, EP-A 608838, EP-A 895809, DE-A 19835247, EP-A 962253, EP-A 1080784, EP-A 1090684, EP-A 1123738, EP-A 1192987, EP-A 1192986, EP-A 1192982, EP-A 1192983 und EP-A 1192988). Nach diesen Verfahren wird von geeigneten Quellen der elementaren Konstituenten der Multimetalloxidmasse ein möglichst inniges, vorzugsweise feinteiliges, Trockengemisch erzeugt und dieses bei Temperaturen von 350 bis 700°C bzw. 400 bis 650°C oder 400 bis 600°C thermisch behandelt. Die thermische Behandlung kann prinzipiell sowohl unter oxidierender, reduzierender als auch unter inerter Atmosphäre erfolgen. Als oxidierende Atmosphäre kommt z.B. Luft, mit molekularem Sauerstoff angereicherte Luft oder an Sauerstoff entreicherte Luft in Betracht. Vorzugsweise wird die thermische Behandlung jedoch unter inerter Atmosphäre, d.h., z.B. unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Erfolgt die thermische Behandlung unter gasförmiger Atmosphäre, kann diese sowohl stehen als auch fließen. Vorzugsweise fließt sie. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (z.B. unter Luft) bei einer Temperatur von 150 bis 400°C bzw. 250 bis 350°C (= Vorzersetzungsschritt). Im Anschluß daran wird die thermische Behandlung zweckmäßig unter Inertgas bei Temperaturen von 350 bis 700°C bzw. 400 bis 650°C oder 450 bis 600°C fortgesetzt. Selbstredend kann die thermische Behandlung auch so erfolgen, daß die Katalysatorvorläufermasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder versplittet wird.

Das innige Vermischen der Ausgangsverbindungen kann in trockener oder in nasser Form erfolgen.

Erfolgt es in trockener Form, werden die Ausgangsverbindungen zweckmäßigerweise als feinteilige Pulver eingesetzt und nach dem Mischen und gegebenenfalls Verdichten der Calcinierung (thermischen Behandlung) unterworfen.

Vorzugsweise erfolgt das innige Vermischen jedoch in nasser Form. Üblicherweise werden die Ausgangsverbindungen dabei in Form einer wäßrigen Lösung (gegebenenfalls unter Mitverwendung komplexbildender Mittel; vgl. z.B. DE-A 10145958) und/oder Suspension miteinander vermischt. Anschließend wird die wäßrige Masse getrocknet und nach der Trocknung calciniert. Zweckmäßigerweise handelt es sich bei der wäßrigen Masse um eine wäßrige Lösung oder um eine wäßrige Suspension. Vorzugsweise erfolgt der Trocknungsprozeß unmittelbar im Anschluß an die Herstellung der wäßrigen Mischung (insbesondere im Fall einer wäßrigen Lösung; vgl. z.B. JP-A 7-315842) und durch Sprühtrocknung (die Austrittstemperaturen betragen in der Regel 100 bis 150°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung oder Suspension handelt. Es kann aber auch durch Eindampfen im Vakuum, durch Gefriertrocknung, oder durch konventionelles Eindampfen getrocknet werden.

Als Quellen für die elementaren Konstituenten kommen im Rahmen der Durchführung der vorstehend beschriebenen Herstellweise von i-/k-Phase-Mischkristall-Multimetalloxidmassen alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/oder Hydroxide der elementaren Konstituenten mitverwendet oder ausschließlich verwendet werden. D.h., insbesondere kommen alle in den Schriften des gewürdigten Standes der Technik genannten Ausgangsverbindungen in Betracht.

Erfindungsgemäß geeignete Quellen für das Element Mo sind z.B. Molybdänoxide wie Molybdäntrioxid, Molybdate wie Ammoniumheptamolybdattetrahydrat und Molybdänhalogenide wie Molybdänchlorid.

Geeignete, erfindungsgemäß mitzuverwendende Ausgangsverbindungen für das Element V sind z.B. Vanadiumoxysulfathydrat, Vanadylacetylacetonat, Vanadate wie Ammoniummetavanadat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Dabei können als Vanadinausgangsverbindungen auch solche mitverwendet werden, die das Vanadin in der Oxidationsstufe +4 enthalten.

Als Quellen für das Element Tellur eignen sich erfindungsgemäß Telluroxide wie Tellurdioxid, metallisches Tellur, Tellurhalogenide wie $TeCl_2$, aber auch Tellursäuren wie Orthotellursäure $H_6TeO_6$.

Vorteilhafte Antimonausgangsverbindungen sind Antimonhalogenide wie $SbCl_3$, Antimonoxide wie Antimontrioxid ($Sb_2O_3$), Antimonsäuren wie $HSb(OH)_6$, aber auch Antimonoxid-Salze wie Antimonoxid-sulfat ($SbO)_2SO_4$.

Erfindungsgemäß geeignete Niobquellen sind z. B. Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Carbonsäuren und/oder Dicarbonsäuren wie z. B. Oxalate und Alkoholate. Selbstredend kommen als Niobquelle auch die in der EP-A 895 809 verwendeten Nb enthaltenden Lösungen in Betracht.

Bezüglich aller anderen möglichen Elemente (insbesondere des Pb, Ni, Cu, Co, Bi und Pd) kommen als geeignete Ausgangsverbindungen vor allem deren Halogenide, Nitrate, Formiate, Oxalate, Acetate, Carbonate und/oder Hydroxide in Betracht. Geeignete Ausgangsverbindungen sind vielfach auch deren Oxoverbindungen wie z. B. Wolframate bzw. die von diesen abgeleiteten Säuren. Häufig werden als Ausgangsverbindungen auch Ammoniumsalze eingesetzt.

Ferner kommen als Ausgangsverbindungen auch Polyanionen vom Anderson Typ in Betracht, wie sie z. B. in Polyhedron Vol. 6, No. 2, pp. 213-218, 1987 beschrieben sind. Eine weitere geeignete Literaturquelle für Polyanionen vom Anderson Typ bildet Kinetics and Catalysis, Vol. 40, No. 3, 1999, pp 401 bis 404.

Andere als Ausgangsverbindungen geeignete Polyanionen sind z. B. solche vom Dawson oder Keggin Typ. Vorzugsweise werden solche Ausgangsverbindungen verwendet, die sich bei erhöhten Temperaturen entweder im Beisein oder bei Ausschluß von Sauerstoff, gegebenenfalls unter Freisetzung gasförmiger Verbindungen, in ihre Oxide umwandeln.

Die wie beschrieben erhältlichen i-/k-Phase-Mischkristall-Multimetalloxidmassen (reine i-Phase Multimetalloxide werden nach der beschriebenen Verfahrensweise allenfalls zufällig erhalten) können dann wie beschrieben durch geeignetes Waschen in erfindungsgemäße Multimetalloxide (I) überführt werden.

Ein erhöhter Anteil an i-Phase (und in günstigen Fällen im wesentlichen reine i-Phase) stellt sich bei der Herstellung von Vorläufermultimetalloxiden (die durch beschriebenes Waschen in erfindungsgemäße Multimetalloxide (I) überführt werden können) dann ein, wenn ihre Herstellung auf hydrothermalem Weg erfolgt, wie es z.B. die DE-A 10029338 und die JP-A 2000-143244 beschreiben.

Die Herstellung von erfindungsgemäßen Multimetalloxidmassen (I) kann aber auch dadurch erfolgen, dass man zunächst eine Multimetalloxidmasse I' erzeugt, die sich von einer Multimetalloxidmasse (I) nur dadurch unterscheidet, dass d = 0 ist.

Eine solche, bevorzugt feinteilige, Multimetalloxidmasse I' kann dann mit Lösungen (z.B. wässrigen) von Elementen $M^3$ getränkt (z.B. durch Besprühen), nachfolgend getrocknet (bevorzugt bei Temperaturen ≤ 100°C) und anschließend wie für die Vorläufermultimetalloxide bereits beschrieben, calciniert (bevorzugt im Inertgasstrom) werden (bevorzugt wird hier auf eine Vorzersetzung an Luft verzichtet) werden. Die Verwendung von wäßrigen Nitrat- und/oder Halogenidlösungen von Elementen $M^3$ und/oder die Verwendung von wässrigen Lösungen in denen die Elemente $M^3$ mit organischen Verbindungen (z.B. Acetate oder Acetylacetonate) komplexiert vorliegen ist für diese Herstellvariante besonders vorteilhaft.

Die wie beschrieben erhältlichen erfindungsgemäßen Multimetalloxide (I) können als solche [z.B. als Pulver oder nach Tablettieren des Pulvers (häufig unter Zusatz von 0,5 bis 2 Gew.-% an feinteiligem Graphit) und nachfolgendem Versplitten zu Splitt zerkleinert] oder auch zu Formkörpern geformt für das erfindungsgemäße Verfahren eingesetzt werden. Dabei kann das Katalysatorbett ein Festbett, ein Wanderbett oder ein Wirbelbett sein.

Die Formung zu Formkörpern kann z.B. durch Aufbringen auf einen Trägerkörper erfolgen, wie es in der DE-A 10118814 bzw. PCT/EP/02/04073 bzw. DE-A 10051419 beschrieben wird.

Die für die erfindungsgemäß einzusetzenden Multimetalloxidmassen (I) zu verwendenden Trägerkörper sind vorzugsweise chemisch inert. D.h., sie greifen in den Ablauf der partiellen katalytischen Gasphasenoxidation bzw.

-ammoxidation des Kohlenwasserstoffs (z.B. Propan und/oder Propen zu Acrylsäure), die durch die erfindungsgemäß zu verwendenden Multimetalloxidmassen (I) katalysiert wird, im wesentlichen nicht ein.

Als Material für die Trägerkörper kommen erfindungsgemäß insbesondere Aluminiumoxid, Siliciumdioxid, Silicate wie Ton, Kaolin, Steatit (bevorzugt mit geringem in Wasser löslichem Alkaligehalt), Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt.

Häufig liegt die Oberflächenrauhigkeit $R_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen $\leq$ 1 Vol.-%).

Die Dicke der auf den erfindungsgemäßen Schalenkatalysatoren befindlichen aktiven Oxidmassenschale liegt üblicherweise bei 10 bis 1000 $\mu$m. Sie kann aber auch 50 bis 700 $\mu$m, 100 bis 600 $\mu$m oder 150 bis 400 $\mu$m betragen. Mögliche Schalendicken sind auch 10 bis 500 $\mu$m, 100 bis 500 $\mu$m oder 150 bis 300 $\mu$m.

Prinzipiell kommen für das erfindungsgemäße Verfahren beliebige Geometrien der Trägerkörper in Betracht. Ihre Längstausdehnung beträgt in der Regel 1 bis 10 mm. Vorzugsweise werden jedoch Kugeln oder Zylinder, insbesondere Hohlzylinder, als Trägerkörper angewendet. Günstige Durchmesser für Trägerkugeln betragen 1,5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung erfindungsgemäß zu verwendender Schalenkatalysatoren kann in einfachster Weise so erfolgen, dass man erfindungsgemäß zu verwendende Oxidmassen der allgemeinen Formel (I) vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse der allgemeinen Formel (I) eine Schicht der Aktivmasse auf der befeuchteten Oberfläche angeheftet. Abschließend wird der beschichtete Trägerkörper getrocknet. Selbstredend kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden katalytisch aktiven Oxidmasse der allgemeinen Formel (I) wird selbstredend an die gewünschte Schalendicke angepaßt.

Für den Schalendickenbereich von 100 bis 500 $\mu$m eignen sich z. B. solche Aktivmassenpulver, von denen wenigstens 50 % der Gesamtzahl der Pulverpartikel ein Sieb der Maschenweite 1 bis 20 $\mu$m passieren und deren numerischer Anteil an Partikeln mit einer Längstausdehnung oberhalb von 50 $\mu$m weniger als 10 % beträgt. In der Regel entspricht die Verteilung der Längstausdehnungen der Pulverpartikel herstellungsbedingt einer Gaußverteilung. Häufig ist die Korngrößenverteilung wie folgt beschaffen:

| D ($\mu$m) | 1 | 1,5 | 2 | 3 | 4 | 6 | 8 | 12 | 16 | 24 | 32 |
|---|---|---|---|---|---|---|---|---|---|---|---|
| x | 80,5 | 76,3 | 67,1 | 53,4 | 41,6 | 31,7 | 23 | 13,1 | 10,8 | 7,7 | 4 |
| y | 19,5 | 23,7 | 32,9 | 46,6 | 58,4 | 68,3 | 77 | 86,9 | 89,2 | 92,3 | 96 |

| D ($\mu$m) | 48 | 64 | 96 | 128 |
|---|---|---|---|---|
| x | 2,1 | 2 | 0 | 0 |
| y | 97,9 | 98 | 100 | 100 |

Dabei sind:

D = Durchmesser des Korns,

x = der prozentuale Anteil der Körner, deren Durchmesser ≥ D ist; und

y = der prozentuale Anteil der Körner, deren Durchmesser < D ist.

Für eine Durchführung des beschriebenen Beschichtungsverfahrens im technischen Maßstab empfiehlt sich z. B. die Anwendung des in der DE-A 2909671, sowie des in der DE-A 10051419 offenbarten Verfahrensprinzips. D.h., die zu beschichtenden Trägerkörper werden in einem vorzugsweise geneigten (der Neigungswinkel beträgt in der Regel ≥ 0° und ≤ 90°, meist ≥ 30° und ≤ 90°; der Neigungswinkel ist der Winkel der Drehbehältermittelachse gegen die Horizontale) rotierenden Drehbehälter (z. B. Drehteller oder Dragiertrommel) vorgelegt. Der rotierende Drehbehälter führt die z. B. kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (z.B. eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (z.B. über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des z. B. zylinder-oder kugelförmigen Trägerkörpers zu einer zusammenhängenden Schale verdichtet.

Bei Bedarf durchläuft der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können usw. (eine Zwischentrocknung ist in der Regel nicht erforderlich). Feinteilige oxidische Aktivmasse und flüssiges Bindemittel werden dabei in der Regel kontinuierlich und simultan zugeführt.

Die Entfernung des flüssigen Bindemittels kann nach beendeter Beschichtung z. B. durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

Wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 2909671 und in der DE-A 10051419.

Die vorerwähnte abschließende Entfernung des verwendeten flüssigen Bindemittels kann in kontrollierter Weise z. B. durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trokkenofen beliebiger Art (z. B. Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden.

Als Bindemittel für den Beschichtungsprozeß können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Von Bedeutung ist, dass die Herstellung erfindungsgemäß geeigneter Schalenkatalysatoren nicht nur durch Aufbringen der fertiggestellten, feingemahlenen aktiven Oxidmassen der allgemeinen Formel (I) auf die befeuchtete Trägerkörperoberfläche erfolgen kann.

Vielmehr kann anstelle der aktiven Oxidmasse auch eine feinteilige Vorläufermasse derselben auf die befeuchtete Trägeroberfläche (unter Anwendung der gleichen Beschichtungsverfahren und Bindemittel) aufgebracht und die Calcination nach Trocknung des beschichteten Trägerkörpers durchgeführt werden (es können auch Trägerkörper mit einer Vorläuferlösung getränkt, nachfolgend getrocknet und anschließend calciniert werden). Abschließend können, falls erforderlich, die von der i-Phase verschiedenen Phasen ausgewaschen werden.

Als eine solche feinteilige Vorläufermasse kommt z. B. diejenige Masse in Betracht, die dadurch erhältlich ist, dass man aus den Quellen der elementaren Konstituenten der gewünschten aktiven Oxidmasse der allgemeinen Formel (I) zunächst ein möglichst inniges, vorzugsweise feinteiliges, Trockengemisch erzeugt (z. B. durch Sprühtrocknung einer wäßrigen Suspension oder Lösung der Quellen) und dieses feinteilige Trockengemisch (gegebenenfalls nach Tablettierung unter Zusatz von 0,5 bis 2 Gew.-% an feinteiligem Graphit) bei einer Temperatur von 150 bis 350°C, vorzugsweise 250 bis 350°C unter oxidierender (Sauerstoff enthaltender) Atmosphäre (z. B. unter Luft) thermisch behandelt (wenige Stunden) und abschließend bei Bedarf einer Mahlung unterwirft.

Nach der Beschichtung der Trägerkörper mit der Vorläufermasse wird dann, bevorzugt unter Inertgasatmosphäre (alle anderen Atmosphären kommen auch in Betracht) bei Temperaturen von 360 bis 700°C bzw. 400 bis 650°C oder 400 bis 600°C calciniert.

Selbstredend kann die Formgebung erfindungsgemäß verwendbarer Multimetalloxidmassen (I) auch durch Extrusion und/oder Tablettierung sowohl von feinteiliger Multimetalloxidmasse (I), als auch von feinteiliger Vorläufermasse einer Multimetalloxidmasse (I) erfolgen (falls erforderlich kann das Auswaschen der von der i-Phase verschiedenen Phasen abschließend erfolgen).

Als Geometrien kommen dabei sowohl Kugeln, Vollzylinder und Hohlzylinder (Ringe) in Betracht. Die Längstausdehnung der vorgenannten Geometrien beträgt dabei in der Regel 1 bis 10 mm. Im Fall von Zylindern beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Vollkatalysatoren können auch eine Länge von 3 bis 6 mm, einen Außendurchmesser von 4 bis 8 mm und eine Wanddicke von 1 bis 2 mm aufweisen. Möglich ist aber auch eine Vollkatalysatorringgeometrie von 7 mm x 3 mm x 4 mm oder von 5 mm x 3 mm x 2 mm (Außendurchmesser x Länge x Innendurchmesser).

Selbstredend kommen für das erfindungsgemäße Verfahren für die Geometrie der zu verwendenden Multimetalloxidaktivmassen (I) auch all jene der DE-A 10101695 in Betracht.

Erfindungsgemäß wesentlich ist, wie bereits gesagt, daß die erfindungsgemäß zu verwendenden Multimetalloxidmassen (I) ein Röntgendiffraktogramm aufweisen (in dieser Schrift stets bezogen auf Cu-$K_\alpha$-Strahlung), das Beugungsreflexe h, i und k aufweist, deren Scheitelpunkte bei den Beugungswinkeln (2Θ) 22,2 $\pm$ 0,4° (h), 27,3 $\pm$ 0,4° (i) und 28,2 $\pm$ 0,4° (k) liegen, wobei

- der Beugungsreflex h innerhalb des Röntgendiffraktogramms der intensitätsstärkste ist, sowie eine Halbwertsbreite von höchstens 0,5° aufweist,

- die Intensität $P_i$ des Beugungsreflexes i und die Intensität $P_k$ des Beugungsreflexes k die Bezeichnung 0,65 $\leq$ R $\leq$ 0,85 erfüllen, in der R das durch die Formel

$$R = P_i/(P_i + P_k)$$

definierte Intensitätsverhältnis ist, und

- die Halbwertsbreite des Beugungsreflexes i und des Beugungsreflexes k jeweils $\leq$ 1° beträgt.

Gleichzeitig soll das Röntgendiffraktogramm keinen Beugungsreflex mit der Scheitelpunktlage 2Θ = 50,0 $\pm$ 0,3° aufweisen.

Die Definition der Intensität eines Beugungsreflexes im Röntgendiffraktogramm bezieht sich in dieser Schrift, wie bereits gesagt, auf die in der DE-A 19835247, sowie die in der DE-A 10051419 und DE-A 10046672 niedergelegte Definition.

D.h., bezeichnet A[1] den Scheitelpunkt eines Reflexes 1 und bezeichnet B[1] in der Linie des Röntgendiffraktogramms bei Betrachtung entlang der zur 2Θ-Achse senkrecht stehenden Intensitätsache das nächstliegende ausgeprägte Minimum (Reflexschultern ausweisende Minima bleiben unberücksichtigt) links vom Scheitelpunkt A[1] und B[2] in entsprechender Weise das nächstliegende ausgeprägte Minimum rechts vom Scheitelpunkt A[1] und bezeichnet C[1] den Punkt, an dem eine vom Scheitelpunkt A[1] senkrecht zur 2Θ-Achse gezogene Gerade eine die Punkte B[1] und B[2] verbindende Gerade schneidet, dann ist die Intensität des Reflexes 1 die Länge des Geradenabschnitts A[1]C[1], der sich vom Scheitelpunkt A[1] zum Punkt C[1] erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der 2Θ Achse und der Intensitätsachse herangezogen werden.

Die Halbwertsbreite ist in dieser Schrift in entsprechender Weise die Länge des Geradenabschnitts, der sich zwischen den beiden Schnittpunkten H[1] und H[2] ergibt, wenn man in der Mitte des Geradenabschnitts A[1]C[1] eine Parallele

zur 2Θ-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen.

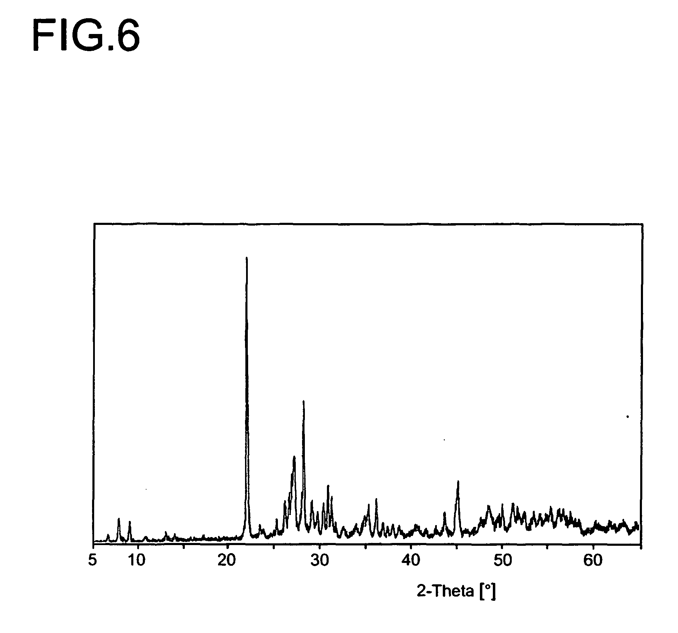

Eine beispielhafte Durchführung der Bestimmung von Halbwertsbreite und Intensität zeigt auch die Figur 6 in der DE-A 10046672.

Selbstredend können die erfindungsgemäß zu verwendenden Multimetalloxidmassen (I) auch in mit feinteiligen, z.B. kolloidalen, Materialien, wie Siliciumdioxid, Titandioxid, Aluminiumoxid, Zirkonoxid, Nioboxid, verdünnter Form als katalytische Aktivmassen eingesetzt werden.

Das Verdünnungsmassenverhältnis kann dabei bis zu 9 (Verdünner): 1 (Aktivmasse) betragen. D.h., mögliche Verdünnungsmassenverhältnisse betragen z.B. 6 (Verdünner): 1 (Aktivmasse) und 3 (Verdünner): 1 (Aktivmasse). Die Einarbeitung der Verdünner kann vor und/oder nach der Calcination, in der Regel sogar vor der Trocknung erfolgen.

Erfolgt die Einarbeitung vor der Trocknung bzw. vor der Calcination, muß der Verdünner so gewählt werden, dass er im fluiden Medium bzw. bei der Calcination im wesentlichen erhalten bleibt. Dies ist z.B. im Fall von bei entsprechend hohen Temperaturen gebrannten Oxiden in der Regel gegeben.

Die erfindungsgemäßen Multimetalloxidmassen (I) eignen sich als solche oder in wie eben beschrieben verdünnter Form als Aktivmassen für heterogen katalysierte partielle Gasphasenoxidationen (einschließlich Oxidehydrierungen) und/oder -ammoxidationen von gesättigten und/oder ungesättigten Kohlenwasserstoffen.

Solche gesättigten und/oder ungesättigten Kohlenwasserstoffe sind insbesondere Ethan, Ethylen, Propan, Propylen, n-Butan, iso-Butan und iso-Buten. Zielprodukte sind dabei vor allem Acrolein, Acrylsäure, Methacrolein, Methacrylsäure, Acrylnitril und Methacrylnitril. Sie eignen sich aber auch für die heterogen katalysierte partielle Gasphasenoxidation und/oder -ammoxidation von Verbindungen wie Acrolein und Methacrolein.

Aber auch Ethylen, Propylen und Essigsäure können Zielprodukt sein.

Unter einer vollständigen Oxidation des Kohlenwasserstoffs wird in dieser Schrift verstanden, dass der im Kohlenwasserstoff insgesamt enthaltene Kohlenstoff in Oxide des Kohlenstoffs ($CO$, $CO_2$) umgewandelt wird.

Alle davon verschiedenen Umsetzungen des Kohlenwasserstoffs unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die partielle Ammoxidation.

Bevorzugt eignen sich die in dieser Schrift niedergelegten erfindungsgemäßen Multimetalloxidmassen (I) als katalytische Aktivmassen für die Umsetzung von Propan zu Acrolein und/oder Acrylsäure, von Propan zu Acrylsäure und/oder Acrylnitril, von Propylen zu Acrolein und/oder Acrylsäure, von Propylen zu Acrylnitril, von iso-Butan zu Methacrolein und/oder Methacrylsäure, von iso-Butan zu Methacrylsäure und/oder Methacrylnitril, von Ethan zu Ethylen, von Ethan zu Essigsäure und von Ethylen zu Essigsäure.

Die Durchführung solcher partiellen Oxidationen und/oder Ammoxidationen (durch in an sich bekannter Weise zu steuernde Wahl des Gehaltes an Ammoniak im Reaktionsgasgemisch kann die Reaktion im wesentlichen ausschließlich als partielle Oxidation, oder ausschließlich als partielle Ammoxidation, oder als Überlagerung beider Reaktionen gestaltet werden; vgl. z.B. WO 98/22421) ist von den i-/k-Phase Mischkristallsystemen des Standes der Technik an sich bekannt und kann in völlig entsprechender Weise durchgeführt werden.

Wird als Kohlenwasserstoff Roh-Propan oder Roh-Propylen eingesetzt, ist dieses bevorzugt wie in der DE-A 10246119 bzw. DE-A 10118814 bzw. PCT/EP/02/04073 beschrieben zusammengesetzt. Ebenso wird bevorzugt wie dort beschrieben verfahren.

Eine mit Multimetalloxid-(I)-aktivmasse-Katalysatoren durchzuführende partielle Oxidation von Propan zu Acrylsäure kann z.B. wie in der EP-A 608838, der WO 0029106, der JP-A 10-36311 und der EP-A 1192987 beschrieben durchgeführt werden.

Als Quelle für den benötigten molekularen Sauerstoff kann z.B. Luft, mit Sauerstoff angereicherte oder an Sauerstoff entreicherte Luft oder reiner Sauerstoff verwendet werden.

Ein solches Verfahren ist auch dann vorteilhaft, wenn das Reaktionsgasausgangsgemisch kein Edelgas, insbesondere kein Helium, als inertes Verdünnungsgas enthält. Im übrigen kann das Reaktionsgasausgangsgemisch neben Propan und molekularem Sauerstoff selbstredend inerte Verdünnungsgase wie z.B. $N_2$, $CO$ und $CO_2$ umfassen. Wasserdampf als Reaktionsgasgemischbestandteil ist erfindungsgemäß vorteilhaft.

D.h., das Reaktionsgasausgangsgemisch, mit dem die erfindugsgemäße Multimetalloxidaktivmasse bei Reaktionstemperaturen von z.B. 200 bis 550°C oder von 230 bis 480°C bzw. 300 bis 440°C und Drükken von 1 bis 10 bar, bzw. 2 bis 5 bar zu belasten ist, kann z.B. nachfolgende Zusammensetzung aufweisen:

1 bis 15, vorzugsweise 1 bis 7 Vol.-% Propan,
44 bis 99 Vol.-% Luft und
0 bis 55 Vol.-% Wasserdampf.

Bevorzugt sind Wasserdampf enthaltende Reaktionsgasausgangsgemische.

Als andere mögliche Zusammensetzungen des Reaktionsgasausgangsgemisches kommen in Betracht:

70 bis 95 Vol.-% Propan,

5 bis 30 Vol.-% molekularer Sauerstoff und

0 bis 25 Vol.-% Wasserdampf.

Selbstredend wird bei einem solchen Verfahren ein Produktgasgemisch erhalten, das nicht ausschließlich aus Acrylsäure besteht. Vielmehr enthält das Produktgasgemisch neben nicht umgesetztem Propan Nebenkomponenten wie Propen, Acrolein, $CO_2$, CO, $H_2O$, Essigsäure, Propionsäure etc., von denen die Acrylsäure abgetrennt werden muß.

Dies kann so erfolgen, wie es von der heterogen katalysierten Gasphasenoxidation von Propen zu Acrylsäure bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierte Kondensation erfolgen, wie es z.B. in der DE-A 19 924 532 beschrieben ist.

Das dabei resultierende wäßrige Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan, welches vorzugsweise in die Gasphasenoxidation rückgeführt wird. Es kann dazu aus dem Restgasgemisch z.B. durch fraktionierte Druckrektifikation teil- oder vollabgetrennt und anschließend in die Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propan bevorzugt zu absorbieren vermag.

Durch nachfolgende Desorption und/oder Strippung mit Luft kann das absorbierte Propan wieder freigesetzt und in das erfindungsgemäße Verfahren rückgeführt werden. Auf diese Weise sind wirtschaftliche Gesamtpropanumsätze erzielbar. Als Nebenkomponente gebildetes Propen wird dabei, ebenso wie bei anderen Abtrennverfahren, in der Regel vom Propan nicht oder nicht vollständig abgetrennt und mit diesem im Kreis geführt. Dies gilt so auch im Fall von anderen homologen gesättigten und olefinischen Kohlenwasserstoffen. Vor allem gilt es ganz generell für erfindungsgemäße heterogen katalysierte partielle Oxidationen und/oder -ammoxidationen von gesättigten Kohlenwasserstoffen.

Dabei macht es sich vorteilhaft bemerkbar, daß die erfindungsgemäßen Multimetalloxidmassen auch die partielle Oxidation und/oder -ammoxidation des homologen olefinischen Kohlenwasserstoffs zum selben Zielprodukt heterogen zu katalysieren vermögen.

So kann mit den erfindungsgemäßen Multimetalloxidmassen (I) als Aktivmassen Acrylsäure durch heterogen katalysierte partielle Gasphasenoxidation von Propen mit molekularem Sauerstoff wie in der DE-A 10118814 bzw. PCT/EP/02/04073 oder der JP-A 7-53448 beschrieben hergestellt werden.

D.h., eine einzige Reaktionszone A ist für die Durchführung des erfindungsgemäßen Verfahrens ausreichend. In dieser Reaktionszone befinden sich als katalytisch aktive Massen ausschließlich Multimetalloxidmasse(I)-Katalysatoren.

Dies ist ungewöhnlich, verläuft die heterogen katalysierte Gasphasenoxidation von Propen zu Acrylsäure doch ganz allgemein in zwei zeitlich aufeinanderfolgenden Schritten. Im ersten Schritt wird üblicherweise Propen im wesentlichen zu Acrolein oxidiert und im zweiten Schritt wird üblicherweise im ersten Schritt gebildetes Acrolein zu Acrylsäure oxidiert.

Konventionelle Verfahren der heterogen katalysierten Gasphasenoxidation von Propen zu Acrylsäure setzen daher üblicherweise für jeden der beiden vorgenannten Oxidationsschritte einen speziellen, auf den Oxidationsschritt maßgeschneiderten, Katalysatortyp ein.

D.h., die konventionellen Verfahren der heterogen katalysierten Gasphasenoxidation von Propen zu Acrylsäure arbeiten im Unterschied zum erfindungsgemäßen Verfahren mit zwei Reaktionszonen.

Selbstredend kann sich beim erfindungsgemäßen Verfahren der Propenpartialoxidation in der einen Reaktionszone A nur ein oder aber auch mehr als ein Multimetalloxidmasse (I) -Katalysator befinden. Natürlich können die erfindungsgemäß einzusetzenden Katalysatoren mit Inertmaterial verdünnt sein, wie es in dieser Schrift beispielsweise auch als Trägermaterial empfohlen wurde.

Längs der einen Reaktionszone A kann beim erfindungsgemäßen Verfahren der Propenpartialoxidation nur eine oder aber auch eine sich längs der Reaktionszone A ändernde Temperatur eines Wärmeträgers zur Temperierung der Reaktionszone A herrschen. Diese Temperaturänderung kann zunehmend oder abnehmend sein.

Wird das erfindungsgemäße Verfahren der Propenpartialoxidation als Festbettoxidation ausgeführt, erfolgt die Durchführung in zweckmäßiger Weise in einem Rohrbündelreaktor, dessen Kontaktrohre mit dem Katalysator beschickt sind. Um die Kontaktrohre wird im Normalfall als Wärmeträger eine Flüssigkeit, in der Regel ein Salzbad geführt.

Mehrere Temperaturzonen längs der Reaktionszone A können dann in einfacher Weise dadurch realisiert werden, daß längs der Kontaktrohre abschnittsweise mehr als ein Salzbad um die Kontaktrohre geführt wird.

Das Reaktionsgasgemisch wird in den Kontaktrohren über den Reaktor betrachtet entweder im Gleichstrom oder im Gegenstrom zum Salzbad geführt. Das Salzbad selbst kann relativ zu den Kontaktrohren eine reine Parallelströmung ausführen. Selbstverständlich kann dieser aber auch eine Querströmung überlagert sein. Insgesamt kann das Salzbad um die Kontaktrohre auch eine mäanderförmige Strömung ausführen, die nur über den Reaktor betrachtet im Gleich- oder im Gegenstrom zum Reaktionsgasgemisch geführt ist.

Die Reaktionstemperatur kann beim erfindungsgemäßen Verfahren der Propenpartialoxidation längs der gesamten Reaktionszone A 200° bis 500°C betragen. Üblicherweise wird sie 250 bis 450°C betragen. Bevorzugt wird die Reaktionstemperatur 330 bis 420°C, besonders bevorzugt 350 bis 400°C betragen.

Der Arbeitsdruck kann beim erfindungsgemäßen Verfahren der Propenpartialoxidation sowohl 1 bar, weniger als 1 bar oder mehr als 1 bar betragen. Erfindungsgemäß typische Arbeitsdrücke sind 1,5 bis 10 bar, häufig 1,5 bis 5 bar.

An das für das erfindungsgemäße Verfahren der Propenpartialoxidation zu verwendende Propen werden keine besonders hohen Ansprüche im Bezug auf seine Reinheit gestellt.

Als Propen kann für ein solches Verfahren, wie bereits gesagt und wie für alle ein- oder zweistufigen Verfahren der heterogen katalysierten Gasphasenoxidation von Propen zu Acrolein und/oder Acrylsäure ganz generell, z.B. Propen (auch Roh-Propen genannt) der nachfolgenden beiden Spezifikationen völlig problemlos verwendet werden:

a) Polymer grade Propylen:

| ≥ 99,6 gew.-% | Propen, |
|---|---|
| ≤ 0,4 gew.-% | Propan, |
| ≤ 300 gew.ppm | Ethan und/oder Methan, |
| ≤ 5 gew.ppm | $C_4$-Kohlenwasserstoffe, |
| ≤ 1 gew.ppm | Acetylen, |
| ≥ 7 gew.ppm | Ethylen, |
| ≤ 5 gew.ppm | Wasser, |
| ≤ 2 gew.ppm | $O_2$, |
| ≤ 2 gew.ppm | Schwefel enthaltende Verbindungen (berechnet als Schwefel), |
| ≤ 1 gew.ppm | Chlor enthaltende Verbindungen (berechnet als Chlor), |
| ≤ 5 gew.ppm | $CO_2$, |
| ≤ 5 gew.ppm | CO, |
| ≤ 10 gew.ppm | Cyclopropan, |
| ≤ 5 gew.ppm | Propadien und/oder Propin, |
| ≤ 10 gew.ppm | $C_{\geq 5}$-Kohlenwasserstoffe und |
| ≤ 10 gew.ppm | Carbonylgruppen enthaltende Verbindungen (be-rechnet als $Ni(CO)_4$). |

b) Chemical grade Propylen:

| ≥ 94 gew.-% | Propen, |
|---|---|
| ≤ 6 gew.-% | Propan, |
| ≤ 0,2 gew.-% | Methan und/oder Ethan, |
| ≤ 5 gew.ppm | Ethylen, |
| ≤ 1 gew.ppm | Acetylen, |
| ≤ 20 gew.ppm | Propadien und/oder Propin, |
| ≤ 100 gew.ppm | Cyclopropan, |
| ≤ 50 gew.ppm | Buten, |

(fortgesetzt)

| $\leq 50$ gew.ppm | Butadien, |
|---|---|
| $\leq 200$ gew.ppm | $C_4$-Kohlenwasserstoffe, |
| $\leq 10$ gew.ppm | $C_{\geq 5}$-Kohlenwasserstoffe, |
| $\leq 2$ gew.ppm | Schwefel enthaltende Verbindungen (berechnet als Schwefel), |
| $\leq 0,1$ gew.ppm | Sulfide (berechnet als $H_2S$), |
| $\leq 1$ gew.ppm | Chlor enthaltende Verbindungen (berechnet als Chlor), |
| $\leq 0,1$ gew.ppm | Chloride (berechnet als $Cl^{\ominus}$) und |
| $\leq 30$ gew.ppm | Wasser. |

Selbstverständlich können alle vorstehend genannten möglichen Begleiter des Propens jeweils aber auch in der zwei- bis zehnfachen der genannten individuellen Menge im Roh-Propen enthalten sein, ohne die Verwendbarkeit des Roh-Propens für das erfindungsgemäße Verfahren bzw. für die bekannten Verfahren der ein- oder zweistufigen heterogen katalysierten Gasphasenoxidation von Propen zu Acrolein und/oder Acrylsäure ganz generell, zu beeinträchtigen.

Dies gilt insbesondere dann, wenn es sich wie bei den gesättigten Kohlenwasserstoffen, dem Wasserdampf, den Kohlenoxiden oder dem molekularen Sauerstoff sowieso um Verbindungen handelt, die entweder als inerte Verdünnungsgase oder als Reaktionspartner in erhöhten Mengen bei den vorgenannten Verfahren am Reaktionsgeschehen teilnehmen. Normalerweise wird das Roh-Propen als solches mit Kreisgas, Luft und/oder molekularem Sauerstoff und/oder verdünnter Luft und/oder Inertgas vermischt für das erfindungsgemäße Verfahren und alle sonstigen Verfahren der heterogen katalysierten Gasphasenoxidation von Propen zu Acrolein und/oder Acrylsäure eingesetzt.

Als Propenquelle kommt für das erfindungsgemäße Verfahren aber auch Propen in Betracht, das im Rahmen eines vom erfindungsgemäßen Verfahren verschiedenen Verfahren als Nebenprodukt gebildet wird und z.B. bis zu 40 % seines Gewichts Propan enthält. Dabei kann dieses Propen zusätzlich noch von anderen, das erfindungsgemäße Verfahren im wesentlichen nicht störenden, Begleitkomponenten begleitet werden.

Als Sauerstoffquelle kann für das erfindungsgemäße Verfahren der Propenpartialoxidation sowohl reiner Sauerstoff als auch Luft oder mit Sauerstoff angereicherte bzw. abgereicherte Luft verwendet werden.

Neben molekularem Sauerstoff und Propen enthält ein für das erfindungsgemäße Verfahren zu verwendendes Reaktionsgasausgangsgemisch üblicherweise noch wenigstens ein Verdünnungsgas. Als solches kommen Stickstoff, Kohlenoxide, Edelgase und niedere Kohlenwasserstoffe wie Methan, Ethan und Propan in Betracht (höhere, z.B. $C_4$-Kohlenwasserstoffe sollten gemieden werden). Häufig wird auch Wasserdampf als Verdünnungsgas verwendet. Vielfach bilden Mischungen aus vorgenannten Gasen das Verdünnungsgas für das erfindungsgemäße Verfahren der partiellen Propenoxidation.

Erfindungsgemäß vorteilhaft erfolgt die erfindungsgemäße heterogen katalysierte Oxidation des Propens im Beisein von Propan.

In typischer Weise ist das Reaktionsgasausgangsgemisch für das erfindungsgemäße Verfahren wie folgt zusammengesetzt (molare Verhältnisse):

Propen : Sauerstoff : $H_2O$ : sonstige Verdünnungsgase = 1 : (0, 1 - 10) : (0 - 70) : (0 - 20).

Vorzugsweise beträgt das vorgenannte Verhältnis 1 : (1-5) : (1 - 40) : (0 - 10).

Wird Propan als Verdünnungsgas verwendet, kann dieses, wie beschrieben, beim erfindungsgemäßen Verfahren vorteilhaft teilweise ebenfalls zu Acrylsäure oxidiert werden.

Erfindungsgemäß vorteilhaft enthält das Reaktionsgasausgangsgemisch molekularen Stickstoff, CO, $CO_2$, Wasserdampf und Propan als Verdünnungsgas.

Das molare Verhältnis von Propan : Propen kann beim erfindungsgemäßen Verfahren folgende Werte annehmen: 0 bis 15, häufig 0 bis 10, vielfach 0 bis 5, zweckmäßig 0,01 bis 3.

Die Belastung der Katalysatorbeschickung mit Propen kann beim erfindungsgemäßen Verfahren der partiellen Propenoxidation z.B. 40 bis 250 Nl/l·h betragen. Die Belastung mit Reaktionsgasausgangsgemisch liegt häufig im Bereich von 500 bis 15000 Nl/l·h, vielfach im Bereich 600 bis 10000 Nl/l·h, häufig 700 bis 5000 Nl/l·h.

Selbstredend wird beim erfindungsgemäßen Verfahren der Propenpartialoxidation zu Acrylsäure ein Produktgasgemisch erhalten, das nicht ausschließlich aus Acrylsäure besteht. Vielmehr enthält das Produktgasgemisch neben nicht umgesetztem Propen Nebenkomponenten wie Propan, Acrolein, $CO_2$, CO, $H_2O$, Essigsäure, Propionsäure etc.,

von denen die Acrylsäure abgetrennt werden muß.

Dies kann so erfolgen, wie es von der heterogen katalysierten zweistufigen (in zwei Reaktionszonen durchgeführten) Gasphasenoxidation von Propen zu Acrylsäure allgemein bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierte Kondensation erfolgen, wie es z.B. in der DE-A 19 924 532 beschrieben ist.

Das dabei resultierende wäßrige Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propen (und gegebenenfalls Propan). Dieses kann aus dem Restgasgemisch z.B. durch fraktionierte Druckrektifikation abgetrennt und anschließend in die erfindungsgemäße Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propen (und gegebenenfalls Propan) bevorzugt zu absorbieren vermag.

Durch nachfolgende Desorption und/oder Strippung mit Luft kann das absorbierte Propen (und gegebenenfalls Propan) wieder freigesetzt und in das erfindungsgemäße Verfahren rückgeführt werden. Auf diese Weise sind wirtschaftliche Gesamtpropenumsätze erzielbar. Wird Propen im Beisein von Propan partialoxidiert, werden Propen und Propan bevorzugt gemeinsam abgetrennt und rückgeführt.

In völlig entsprechender Weise lassen sich die erfindungsgemäßen Multimetalloxide (I) als Katalysatoren für die Partialoxidation von iso-Butan und/oder iso-Buten zu Methacrylsäure einsetzen.

Ihre Verwendung für die Ammoxidation von Propan und/oder Propen kann z.B. wie in der EP-A 529853, der DE-A 2351151, der JP-A 6-166668 und der JP-A 7-232071 beschrieben erfolgen.

Ihre Verwendung für die Ammoxidation von n-Butan und/oder n-Buten kann wie in der JP-A 6-211767 beschrieben erfolgen.

Ihre Verwendung für die Oxidehydrierung von Ethan zu Ethylen, bzw. die Weiterreaktion zu Essigsäure, kann wie in der US-A 4250346 oder wie in der EP-B 261264 beschrieben erfolgen.

Die erfindungsgemäßen Multimetalloxidmassen (I) können aber auch in andere Multimetalloxidmassen integriert werden (z.B. ihre feinteiligen Massen vermengen, gegebenenfalls verpressen und calcinieren, oder als Schlämmen (vorzugsweise wässrig) vermengen, trocknen und calcinieren (z.B. wie es die EP-A 529853 für Multimetalloxidmassen (I) mit d = 0 beschreibt)). Bevorzugt wird wieder unter Inertgas calciniert.

Die dabei resultierenden Multimetalloxidmassen (nachfolgend Gesamtmassen genannt) enthalten bevorzugt ≥ 50 Gew.-%, besonders bevorzugt ≥ 75 Gew.-%, und ganz besonders bevorzugt ≥ 90 Gew.-% bzw. ≥ 95 Gew.-% an Multimetalloxidmassen (I) und sind für die in dieser Schrift besprochenen Partialoxidationen und/oder -ammoxidationen ebenfalls geeignet.

Bevorzugt enthalten auch die Gesamtmassen bei $2\Theta = 50{,}0 \pm 0{,}3°$ keine Beugungsreflex-Scheitelpunktlage.

Enthält die Gesamtmasse bei $2\Theta = 50{,}0 \pm 0{,}3°$ eine Beugungsrelfex-Scheitelpunktlage, ist es günstig, wenn der Gewichtsanteil der erfindungsgemäßen Multimetalloxidmassen (I) ≥ 80 Gew.-%, oder ≥ 90 Gew.-%, oder ≥ 95 Gew.-% beträgt. Solche Gesamtmassen sind z.B. dadurch erhältlich, dass beim erfindungsgemäßen Herstellverfahren für die Multimetalloxidmassen (I) nicht quantitativ ausgewaschen wird.

Die geometrische Formgebung erfolgt bei den Gesamtmassen in zweckmäßiger Weise wie für die Multimetalloxidmassen (I) beschrieben.

Die Vorteilhaftigkeit der erfindungsgemäßen Multimetalloxidmassen (I) basiert auf ihrer hervorragenden Zielproduktselektivität. Es überrascht, dass die Promotoren $M^3$ auch in reiner i-Phase wirksam sind, und zwar sowohl bezüglich der in der Schrift genannten Partialoxidationen als auch Partialammoxidationen.

Zum Zweck der heterogen katalysierten partiellen Gasphasenoxidation von Propan zu Acrylsäure werden die erfindungsgemäßen Multimetalloxidmassen (I) und diese enthaltende Multimetalloxidmassen bzw. Katalysatoren bevorzugt wie in der DE-A 10122027 beschrieben in Betrieb genommen.

Beispiele

A) Herstellung von Multimetalloxidmassen-Schalenkatalysatoren

Vergleichsbeispiel 1 (Herstellung eines Multimetalloxidkatalysators mit der Aktivmasse $Mo_{1,0}V_{0,33}Te_{0,19}Nb_{0,11}Ni_{0,01}O_x$, enthaltend i-und k-Phase)

87,61 g Ammoniummetavanadat (78,55 Gew.-% $V_2O_5$, Fa. G.f.E. Nürnbern, DE) wurden bei 80°C in 3040 ml Wasser (Dreihalskolben mit Rührer, Thermometer, Rückflußkühler und Heizung) unter Rühren gelöst. Es entstand eine klare, gelbliche Lösung. Diese Lösung wurde auf 60°C abgekühlt und dann unter Aufrechterhaltung der 60°C in der genannten Reihenfolge nacheinander 117,03 g Tellursäure (99 Gew.-% $H_6TeO_6$, Fa. Aldrich) und 400,00 g Ammoniumheptamolybdat (82,52 Gew.-% $MoO_3$, Fa. Starck/Goslar) in die Lösung eingerührt. Die resultierende tiefrote Lösung wurde auf 30°C abgekühlt und dann unter Aufrechterhaltung der 30°C mit 25,60 g einer wäßrigen Lösung von 6,80 g Nickel-(II)-nitrat Hexahydrat (98 Gew.-%, Fa. Fluka) in 20 g Wasser (Lösung erfolgte bei 25°C) versetzt. Es wurde so eine Lösung A erhalten, die 30°C aufwies.

In einem Becherglas wurden davon getrennt bei 60°C 112,67 g Ammoniumnioboxalat (20,8 Gew.-% Nb, Fa. Starck/Goslar) in 500 ml Wasser unter Erhalt einer Lösung B gelöst. Lösung B wurde auf 30°C abgekühlt und bei dieser Temperatur mit der dieselbe Temperatur aufweisenden Lösung A vereinigt, wobei die Lösung B zur Lösung A gegeben wurde. Die Zugabe erfolgte stetig über einen Zeitraum von 5 min.. Es entstand eine orangefarbene Suspension.

Diese Suspension wurde anschließend in einem Sprühtrockner der Fa. Niro (Sprühtrockner Niro A/S Atomizer, Transportable Minor Anlage, Zentrifugalzerstäuber der Fa. Niro, DK) sprühgetrocknet. Die Vorlagetemperatur betrug 30°C. Die Gaseintrittstemperatur $T^{ein}$ betrug 320°C, die Gasaustrittstemperatur $T^{aus}$ betrug 110°C. Das resultierende Sprühpulver war ebenfalls orangefarben.

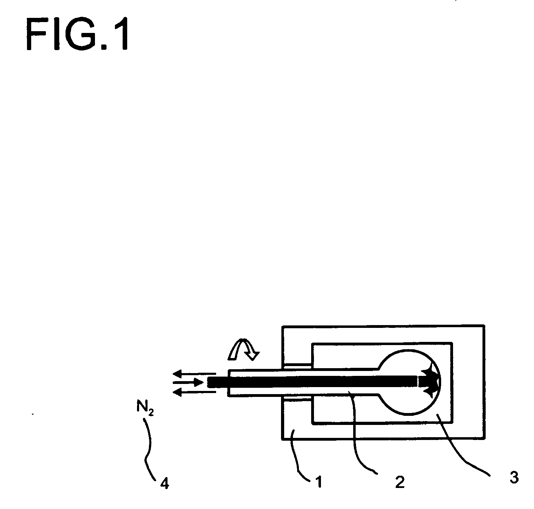

100 g des Sprühpulvers wurden in einem Drehkugelofen gemäß Figur 1 (Quarzglaskugel mit 1 Liter Innenvolumen; 1 = Ofengehäuse, 2 = Drehkolben, 3 = beheizter Raum, 4 = Stickstoff-/Luftstrom) unter einem Luftstrom von 50 Nl/h innerhalb von 27,5 min. zunächst linear von 25°C auf 275°C aufgeheizt und diese Temperatur und der Luftstrom anschließend für 1 h aufrechterhalten. Unmittelbar daran anschließend wurde der Luftstrom durch einen Stickstoffstrom von 50 Nl/h ersetzt und innerhalb von 32,5 min. linear von 275°C auf 600°C aufgeheizt. Diese Temperatur und der Stickstoffstrom wurden dann während 2 h aufrechterhalten. Abschließend wurde unter Aufrechterhaltung des Stickstoffstromes der gesamte Drehkugelofen auf 25°C abgekühlt.

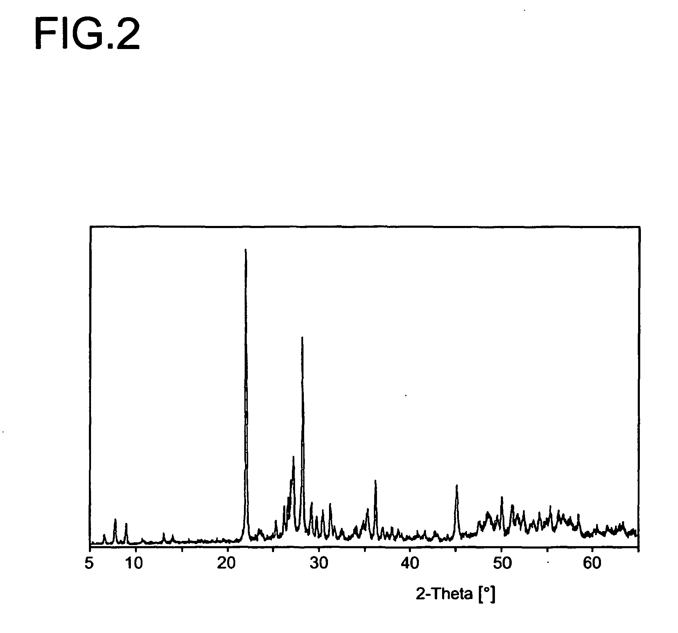

Es wurde ein schwarzes Pulver der Zusammensetzung $Mo_{1,0}V_{0,33}Te_{0,19}Nb_{0,11}Ni_{0,01}O_x$ (Einwaagestöchiometrie: $Mo_{1,0}V_{0,33}Te_{0,22}Nb_{0,11}Ni_{0,01}Ox$) erhalten. Das zugehörige Röntgendiffraktogramm zeigt die Figur 2 (R = 0,26). BET = 8,0 $m^2$/g.

Das Aktivmassenpulver wurde anschließend in einer Retsch-Mühle (Zentrifugalmühle, Typ ZM 100, Fa. Retsch, DE) gemahlen (Korngröße ≤ 0,12 mm).

38 g des nach Mahlung vorliegenden Pulvers wurde auf 150 g kugelförmige Trägerkörper mit einem Durchmesser von 2,2 bis 3,2 mm ($R_z$ = 45 μm, Trägermaterial = Steatit der Fa. Ceramtec, DE, Porengesamtvolumen des Trägers ≤ 1 öl.-% bezogen auf das Trägergesamtvolumen) aufgebracht. Dazu wurde der Träger in eine Dragiertrommel mit 2 1 Innenvolumen (Neigungswinkel der Trommelmittelachse gegen die Horizontale = 30°) vorgelegt. Die Trommel wurde mit 25 Umdrehungen je Minute in Rotation versetzt. Über eine mit 300 Nl/h Druckluft betriebene Zerstäuberdüse wurden über 60 min. hinweg ca. 25 ml eines Gemisches aus Glycerin und Wasser (Gewichtsverhältnis Glycerin: Wasser = 1:3) auf den Träger gesprüht. Die Düse war dabei derart installiert, daß der Sprühkegel die in der Trommel durch Mitnahmebleche an den obersten Punkt der geneigten Trommel beförderten Trägerkörper in der oberen Hälfte der Abrollstrecke benetzte. Das feinteilige Aktivmassenpulver wurde über eine Pulverschnecke in die Trommel eingetragen, wobei der Punkt der Pulverzugabe innerhalb der Abrollstrecke oder unterhalb des Sprühkegels lag. Durch die periodische Wiederholung von Benetzung und Pulveraufdosierung wurde der grundbeschichtete Trägerkörper in der darauffolgenden Periode selbst zum Trägerkörper.

Nach Abschluß der Beschichtung wurde der beschichtete Trägerkörper unter Luft während 16 h bei 150°C im Muffelofen getrocknet. Es resultierte ein Schalenkatalysator VB1 mit 20 Gew.-% Aktivmassenanteil.

Beispiel 1

Wie das Vergleichsbeispiel 1. Das nach der Mahlung in der Retsch-Mühle resultierende Pulver wurde jedoch in 1000 ml einer 10 gew.-%igen wässrigen $HNO_3$-Lösung für 7 h bei 70°C unter Rückfluß gerührt. Der dabei verbliebene Feststoff wurde aus der resultierenden Aufschlämmung abfiltriert und mit Wasser nitratfrei gewaschen. Anschließend wurde der Filterkuchen über Nacht unter Luft bei 110°C in einem Muffelofen getrocknet.

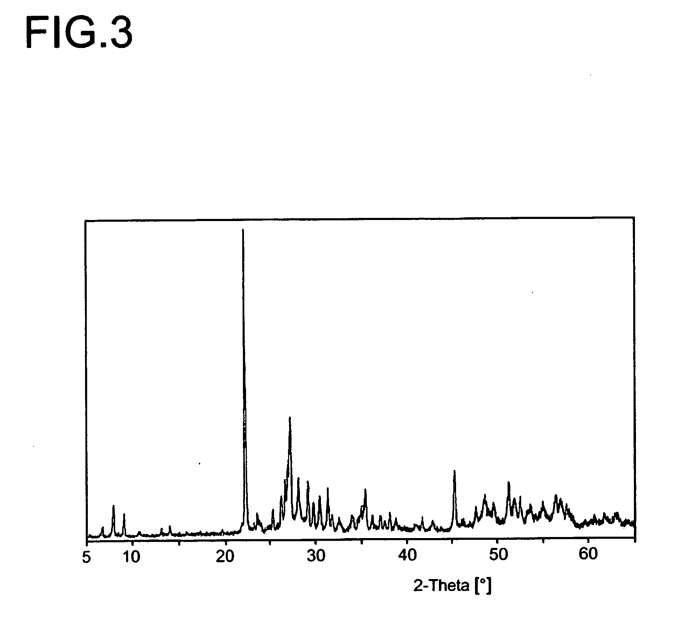

Die resultierende Aktivmasse hatte die Zusammensetzung $Mo_{1,0}V_{0,29}Te_{0,14}Nb_{0,13}Ni_{0,007}O_x$. Das zugehörige Röntgendiffraktogramm zeigt Figur 3 (R = 0,71). BET = 20,2 $m^2$/g.

Sie wurde in gleicher Weise auf denselben Träger wie in Vergleichsbeispiel 1 aufgebracht, so dass ein Schalenkatalysator B1 mit 20 Gew.-% Aktivmassenanteil resultierte.

Vergleichsbeispiel 2

Wie Vergleichsbeispiel 1, anstelle von 6,80 g Nickel-(II)-nitrat Hexahydrat wurden jedoch 6,17 g Palladium-(II)-nitrat Dihydrat 5 (98 %, Fa. Fluka) verwendet.

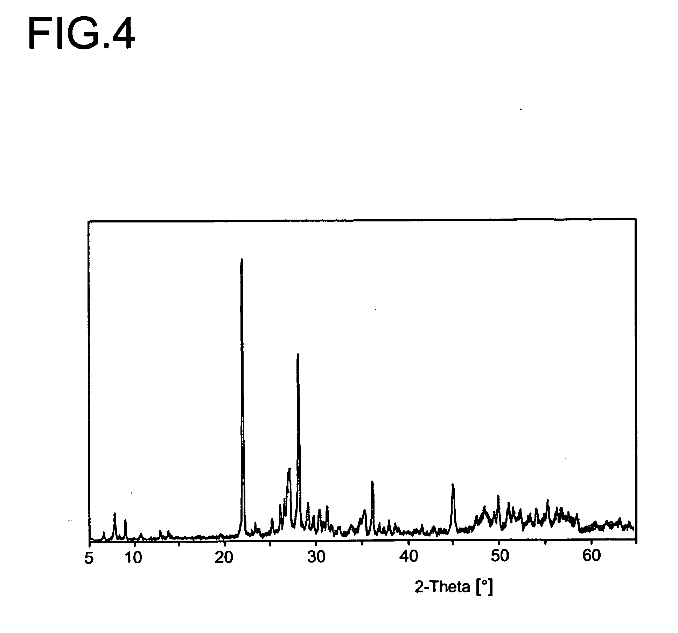

Die resultierende Aktivmasse wies die Zusammensetzung $Mo_{1,0}V_{0,33}Te_{0,19}Nb_{0,11}Pd_{0,01}O_x$ auf. Das zugehörige Röntgendiffraktogramm zeigt Figur 4 (R = 0,25). BET = 9,3 m$^2$/g. Sie wurde in gleicher Weise auf denselben Träger wie in Vergleichsbeispiel 1 aufgebracht, so dass ein Schalenkatalysator VB2 mit 20 Gew.-% Aktivmassenanteil resultierte.

Beispiel 2

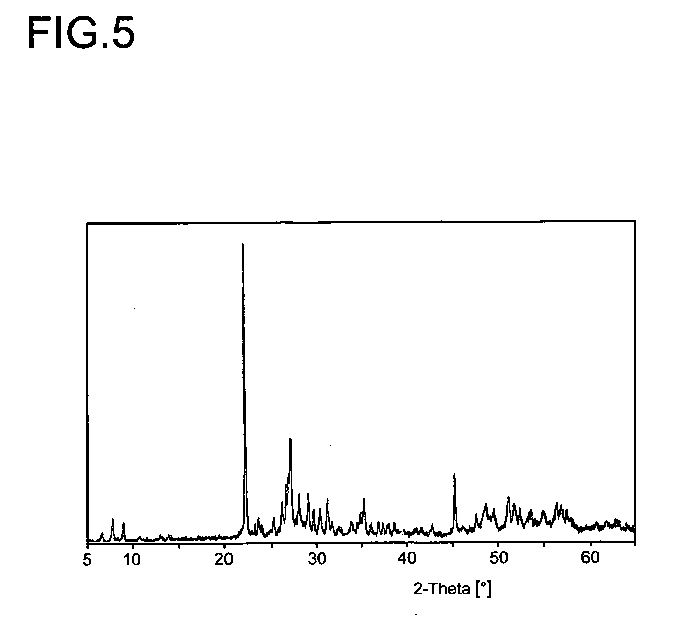

Wie Beispiel 1, es wurde jedoch die Aktivmasse aus Vergleichsbeispiel 2 mit wäßriger Salpetersäure gewaschen. Die resultierende Aktivmasse hatte die Zusammensetzung $Mo_{1,0}V_{0,28}Te_{0,13}Nb_{0,13}Pd_{0,001}O_x$.
Das zugehörige Röntgendiffraktogramm zeigt Figur 5 (R = 0,73). BET = 22,5 m$^2$/g. Sie wurde in gleicher Weise auf denselben Träger wie in Vergleichsbeispiel 1 aufgebracht, so daß ein Schalenkatalysator B2 mit 20 Gew.-% Aktivmassenanteil resultierte.

Vergleichsbeispiel 3

Wie Vergleichsbeispiel 1, der Ansatz wurde jedoch in seiner Menge halbiert ausgeführt und anstelle von dann 3,40 g Nickel-(II)-nitrat Hexahydrat wurden 12,34 g Palladium-(II)-nitrat Dihydrat (98 %, Fa. Fluka) verwendet.
Die resultierende Aktivmasse wies die Zusammensetzung $Mo_{1,0}V_{0,33}Te_{0,22}Nb_{0,11}Pd_{0,04}O_x$ auf. Das zugehörige Röntgendiffraktogramm zeigt Figur 6 (R = 0,35). BET = 9,3 m$^2$/g. Sie wurde in gleicher Weise auf denselben Träger wie in Vergleichsbeispiel 1 aufgebracht, so dass ein Schalenkatalysator VB3 mit 20 Gew.-% Aktivmassenanteil resultierte.

Beispiel 3

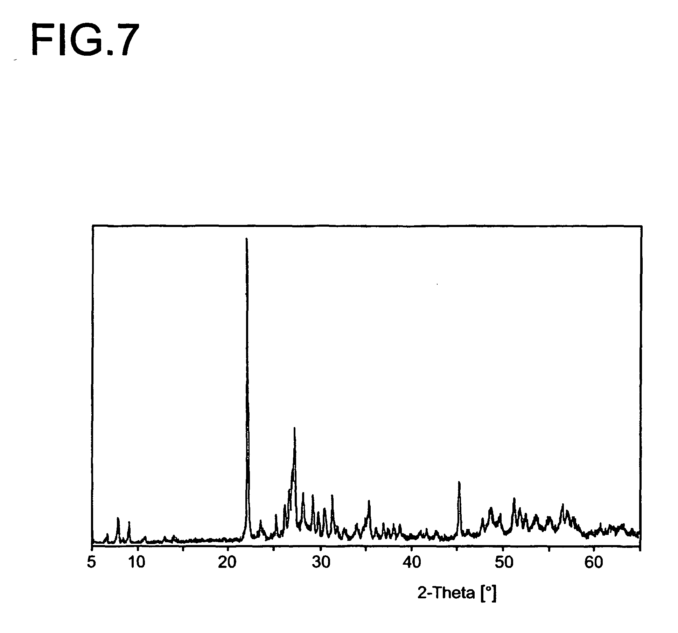

Wie Beispiel 1, es wurde jedoch die Aktivmasse aus Vergleichsbeispiel 3 mit wäßriger Salpetersäure gewaschen. Die resultierende Aktivmasse hatte die Zusammensetzung $Mo_{1,0}V_{0,29}Te_{0,13}Nb_{0,13}Pd_{0,001}O_x$. Das zugehörige Röntgendiffraktogramm zeigt Figur 7 (R = 0,74). BET = 17,4 m$^2$/g. Sie wurde in gleicher Weise auf denselben Träger wie in Vergleichsbeispiel 1 aufgebracht, so daß ein Schalenkatalysator B2 mit 20 Gew.-% Aktivmassenanteil resultierte.

Vergleichsbeispiel 4

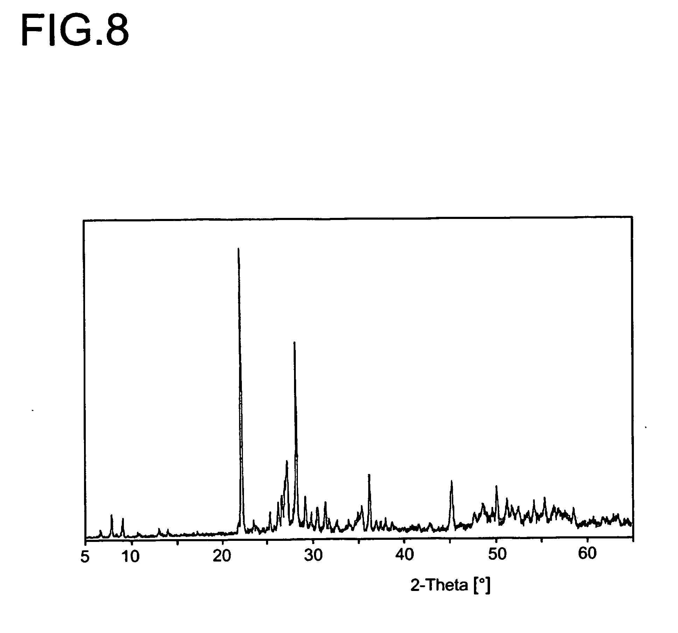

Wie Vergleichsbeispiel 1, anstelle von 6,80 g Nickel-(II)-nitrat Hexahydrat wurden jedoch 3,41 g Cobalt-(II)-nitrat Hexahydrat (98 %, Fa. Riedel-de-Haen) verwendet.
Die resultierende Aktivmasse wies die Zusammensetzung $Mo_{1,0}V_{0,33}Te_{0,19}Nb_{0,11}Co_{0,005}O_x$ auf. Das zugehörige Röntgendiffraktogramm zeigt Figur 8 (R = 0,24). BET = 8,9 m$^2$/g. Sie wurde in gleicher Weise auf denselben Träger wie in Vergleichsbeispiel 1 aufgebracht, so daß ein Schalenkatalysator VB4 mit 20 Gew.-% Aktivmassenanteil resultierte.

Beispiel 4

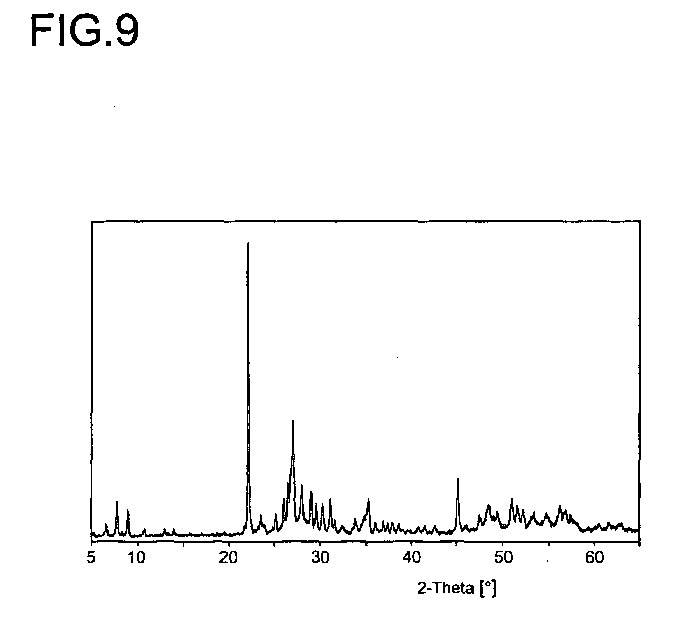

Wie Beispiel 1, es wurde jedoch die Aktivmasse aus Vergleichsbeispiel 4 mit wäßriger Salpetersäure gewaschen.
Die resultierende Aktivmasse hatte die Zusammensetzung $Mo_{1,0}V_{0,29}Te_{0,13}Nb_{0,13}Co_{0,004}O_x$. Das zugehörige Röntgendiffraktogramm zeigt Figur 9 (R = 0,73). BET = 24,6 m$^2$/g. Sie wurde in gleicher Weise auf denselben Träger wie in Vergleichsbeispiel 1 aufgebracht, so daß ein Schalenkatalysator B4 mit 20 Gew.-% Aktivmassenanteil resultierte.

Vergleichsbeispiel 5

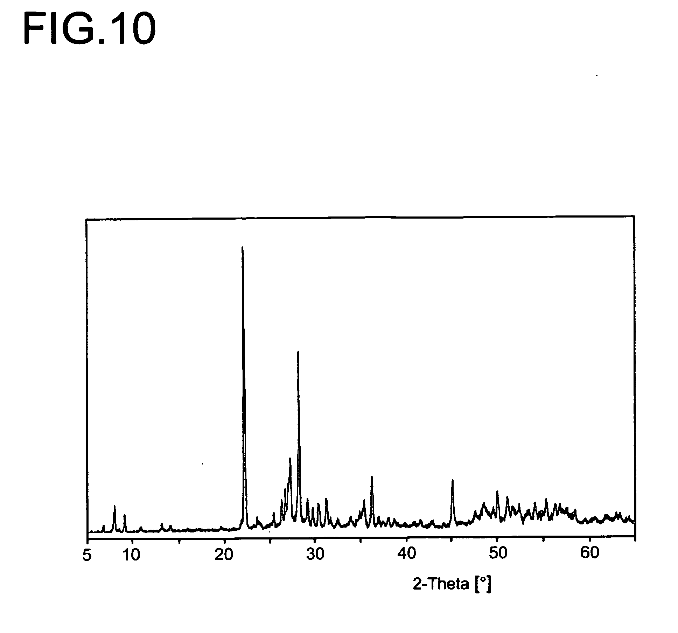

Wie Vergleichsbeispiel 1, anstelle von 6,80 g Nickel-(II)-nitrat Hexahydrat wurden jedoch 5,65 g Kupfer-(II)-nitrat Trihydrat (99 %, Fa. Acros Organics) verwendet.
Die resultierende Aktivmasse wies die Zusammensetzung $Mo_{1,0}V_{0,33}Te_{0,19}Nb_{0,11}Cu_{0,01}O_x$ auf. Das zugehörige Röntgendiffraktogramm zeigt Figur 10 (R = 0,27). BET = 6,7 m$^2$/g. Sie wurde in gleicher Weise auf denselben Träger wie in Vergleichsbeispiel 1 aufgebracht, so dass ein Schalenkatalysator VB5 mit 20 Gew.-% Aktivmassenanteil resultierte.

Beispiel 5

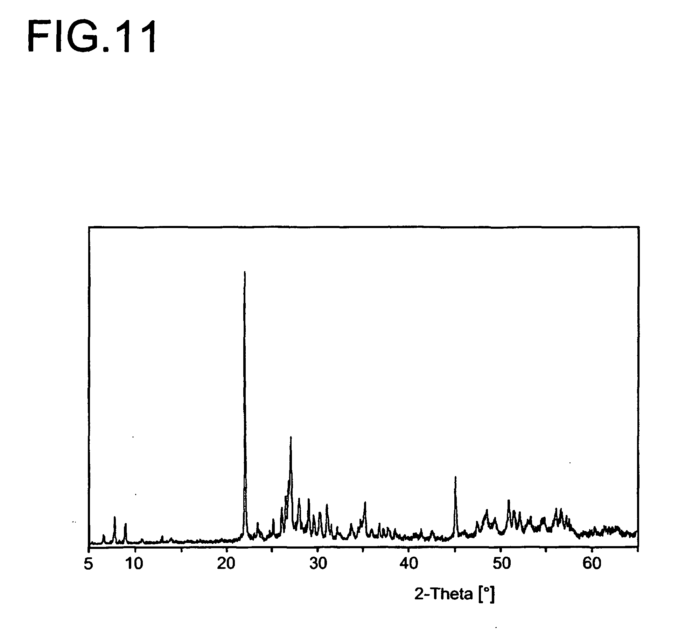

Wie Beispiel 1, es wurde jedoch die Aktivmasse aus Vergleichsbeispiel 5 mit wäßriger Salpetersäure gewaschen.
Die resultierende Aktivmasse hatte die Zusammensetzung $Mo_{1,0}V_{0,28}Te_{0,13}Nb_{0,13}Cu_{0,003}O_x$. Das zugehörige Röntgendiffraktogramm zeigt Figur 11 (R = 0,74). BET = 23,1 m$^2$/g. Sie wurde in gleicher Weise auf denselben Träger wie in Vergleichsbeispiel 1 aufgebracht, so daß ein Schalenkatalysator B5 mit 20 Gew.-% Aktivmassenanteil resultierte.

Vergleichsbeispiel 6

Wie Vergleichsbeispiel 1, anstelle von 6,80 g Nickel-(II)-nitrat Hexahydrat wurden jedoch 5,68 g Wismut-(III)-nitrat Pentahydrat (98,5 %, Fa. Merck) verwendet.

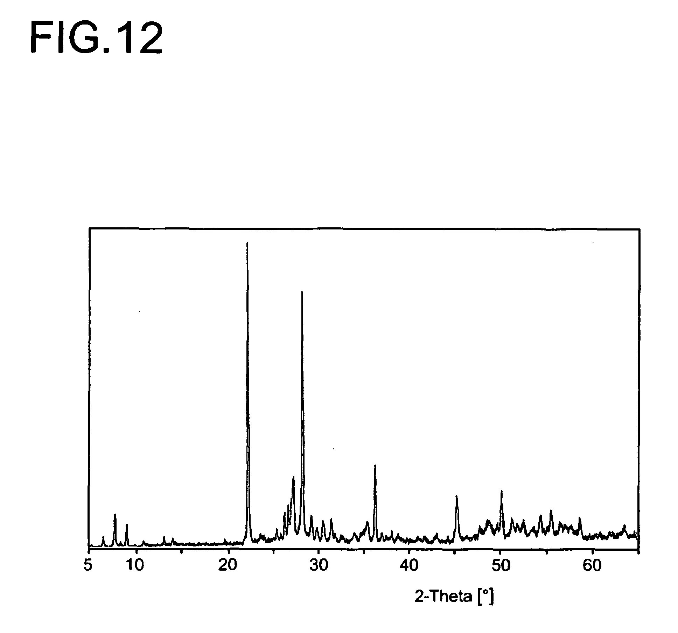

Die resultierende Aktivmasse wies die Zusammensetzung $Mo_{1,0}V_{0,33}Te_{0,19}Nb_{0,11}Bi_{0,004}O_x$ auf. Das zugehörige Röntgendiffraktogramm zeigt Figur 12 (R = 0,18). BET = 9,0 $m^2$/g. Sie wurde in gleicher Weise auf denselben Träger wie in Vergleichsbeispiel 1 aufgebracht, so daß ein Schalenkatalysator VB6 mit 20 Gew.-% Aktivmassenanteil resultierte.

Beispiel 6

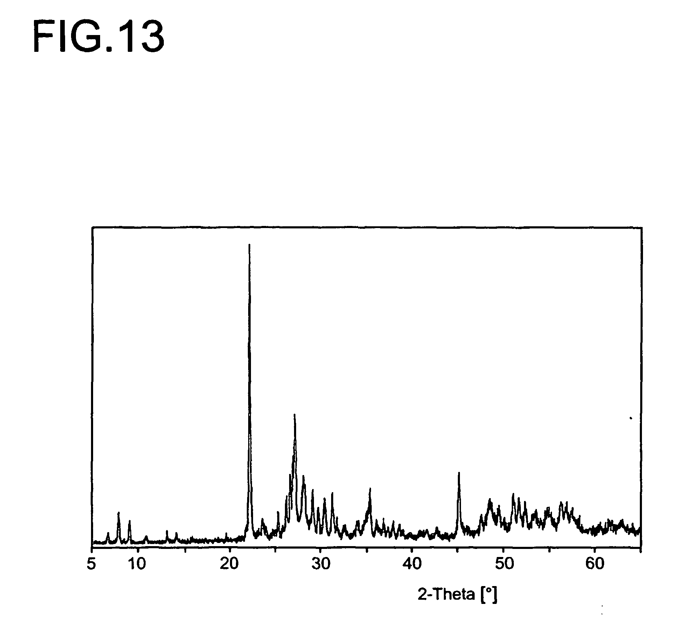

Wie Beispiel 1, es wurde jedoch die Aktivmasse aus Vergleichsbeispiel 6 mit wäßriger Salpetersäure gewaschen. Die resultierende Aktivmasse hatte die Zusammensetzung $Mo_{1,0}V_{0,28}Te_{0,15}Nb_{0,14}Bi_{0,005}O_x$. Das zugehörige Röntgendiffraktogramm zeigt Figur 13 (R = 0,70). BET = 22,0 $m^2$/g. Sie wurde in gleicher Weise auf denselben Träger wie in Vergleichsbeispiel 1 aufgebracht, so daß ein Schalenkatalysator B6 mit 20 Gew.-% Aktivmassenanteil resultierte.

Vergleichsbeispiel 7

Wie Vergleichsbeispiel 1, anstelle von 6,80 g Nickel-(II)-nitrat Hexahydrat wurden jedoch 3,84 g Blei-(II)-nitrat (Riedel-de-Haen, 99 %) verwendet.

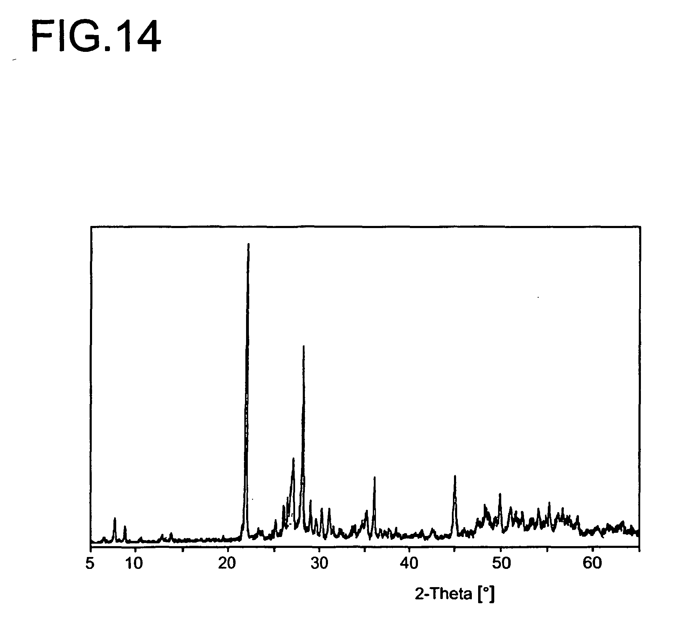

Die resultierende Aktivmasse wies die Zusammensetzung $Mo_{1,0}V_{0,34}Te_{0,18}Nb_{0,11}Pb_{0,004}$ auf. Das zugehörige Röntgendiffraktogramm zeigt Figur 14 (R = 0,30). BET = 2,2 $m^2$/g. Sie wurde in gleicher Weise auf denselben Träger wie in Vergleichsbeispiel 1 aufgebracht, so daß ein Schalenkatalysator VB7 mit 20 Gew.-% Aktivmassenanteil resultierte.

Beispiel 7

Wie Beispiel 1, es wurde jedoch die Aktivmasse aus Vergleichsbeispiel 7 mit wäßriger Salpetersäure gewaschen. Die resultierende Aktivmasse hatte die Zusammensetzung $Mo_{1,0}V_{0,28}Te_{0,13}Nb_{0,13}Pb_{0,001}O_x$.

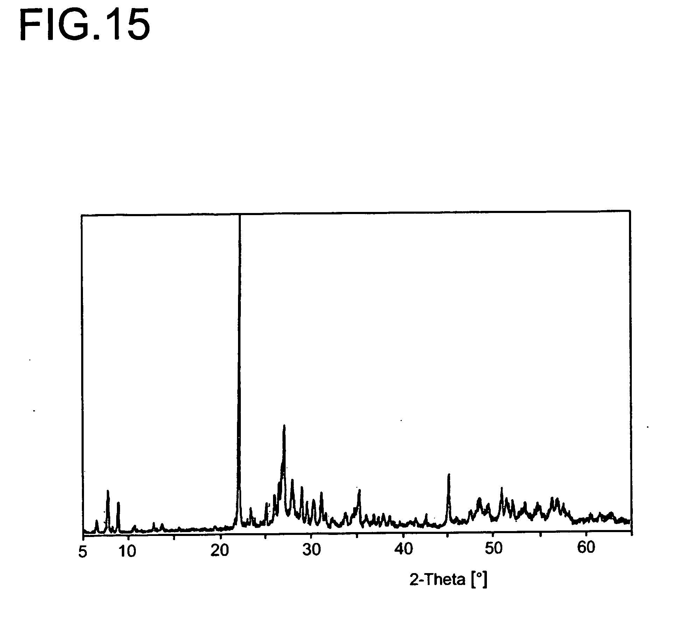

Das zugehörige Röntgendiffraktogramm zeigt Figur 15 (R = 0,67). BET = 27,1 $m^2$/g. Sie wurde in gleicher Weise auf denselben Träger wie in Vergleichsbeispiel 1 aufgebracht, so daß ein Schalenkatalysator B7 mit 20 Gew.-% Aktivmassenanteil resultierte.

Vergleichsbeispiel 8

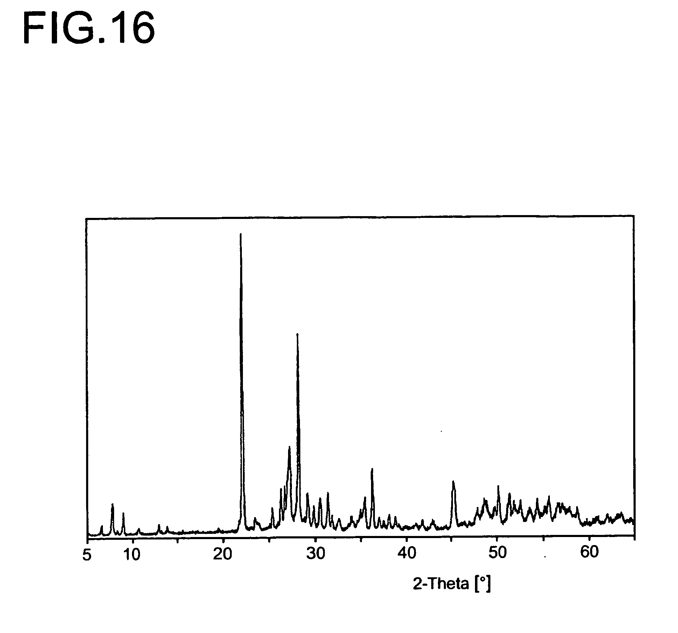

Wie Vergleichsbeispiel 1, jedoch mit dem Unterschied, dass der Zusatz der 5,60 g Nickel-(II)-nitrat Hexahydrat nicht vorgenommen wurde. Die resultierende Aktivmasse wies die Zusammensetzung $Mo_{1,0}V_{0,33}Te_{0,16}Nb_{0,11}O_x$ auf. Das zugehörige Röntgendiffraktogramm zeigt Figur 16 (R = 0,26). BET = 6,7 $m^2$/g. Sie wurde in gleicher Weise auf denselben Träger wie in Vergleichsbeispiel 1 aufgebracht, so daß ein Schalenkatalysator VB8 mit 20 Gew.-% Aktivmassenanteil resultierte.

Vergleichsbeispiel 9

Wie Beispiel 1, es wurde jedoch die Aktivmasse aus Vergleichsbeispiel 7 mit wäßriger Salpetersäure gewaschen. Die resultierende Aktivmasse hatte die Zusammensetzung $Mo_{1,0}V_{0,29}Te_{0,13}Nb_{0,13}O_x$.

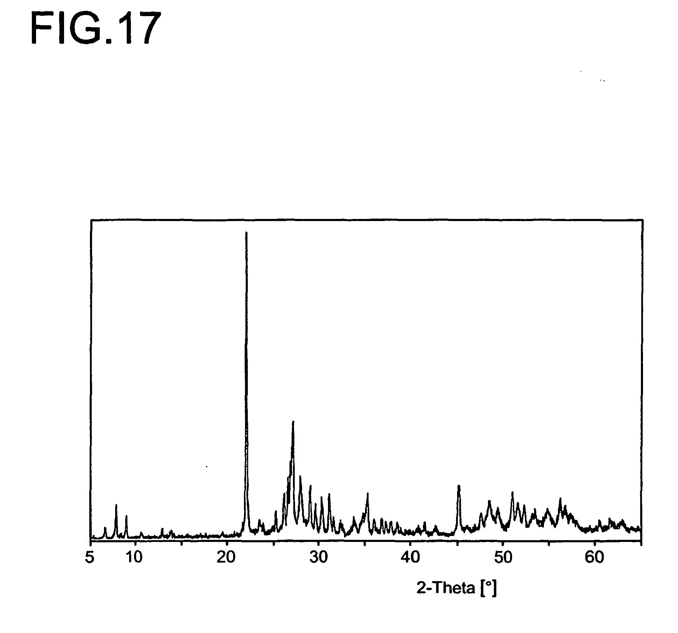

Das zugehörige Röntgendiffraktogramm zeigt Figur 17 (R = 0,68). BET = 26,0 $m^2$/g. Sie wurde in gleicher Weise auf denselben Träger wie in Vergleichsbeispiel 1 aufgebracht, so daß ein Schalenkatalysator VB9 mit 20 Gew.-% Aktivmassenanteil resultierte.

B) Testung der in A) hergestellten Multimetalloxidmassen-Schalenkatalysatoren

Mit jeweils 35,0 g des jeweiligen Schalenkatalysators aus A) wurde ein aus Stahl gefertigter Rohrreaktor (Innendurchmesser: 8,5 mm, Länge: 140 cm, Wanddicke: 2,5 cm) beschickt (Katalysatorschüttlänge in allen Fällen ca. 53 cm). Vor der Katalysatorschüttung wurde eine Vorschüttung von 30 cm Steatitkugeln (Durchmesser: 2,2 bis 3,2 mm, Hersteller: Fa. Ceramtec) und nach der Katalysatorschüttung auf der Restlänge des Rohrreaktors eine Nachschüttung derselben Steatitkugeln angebracht.

Mittels elektrisch beheizter Heizmatten wurde von außen die Außentemperatur des beschickten Reaktionsrohres auf der gesamten Länge auf 350°C eingestellt.

Dann wurde das Reaktionsrohr mit einem Reaktionsgasausgangsgemisch der molaren Zusammensetzung Propan: Luft: $H_2O$ = 1:15:14 beschickt (die Eintrittsseite war auf der Seite der Nachschüttung). Die Verweilzeit (bezogen auf das Katalysatorschüttungsvolumen) wurde auf 2,4 sec. eingestellt. Der Eingangsdruck betrug 2 bar absolut.

Die Reaktionsrohrbeschickung wurde zunächst jeweils bei der vorgenannten Außentemperatur des beschickten Reaktionsrohres über einen Zeitraum von 24 h eingefahren, bevor diese Außentemperatur so erhöht wurde, daß, bezogen auf einmaligen Reaktionsrohrdurchgang, in allen Fällen ein Umsatz des Propans ($U_{PAN}$) von etwa 78 mol-% resultierte.

Die nachfolgende Tabelle zeigt in Abhängigkeit vom verwendeten Schalenkatalysator die für diesen Umsatz benötigte Außentemperatur T (°C), sowie die dabei resultierende Selektivität der Acrylsäurebildung ($S_{ACS}$ (mol-%)) und die Selektivität der Nebenproduktbildung an Propen ($S_{PEN}$ (mol-%)). Zusätzlich zeigt die Tabelle das Intensitätsverhältnis R der auf dem Schalenkatalysator befindlichen Aktivmasse und die Zusammensetzung dieser Aktivmasse.

Tabelle

| Beispiel | Zusammensetzung | R | T [°C] | $U_{PAN}$ (mol-%) | $S_{ACS}$ (mol-%) | $S_{PEN}$ (mol-%) |
|---|---|---|---|---|---|---|
| VB1 | $Mo_1V_{0,33}Te_{0,22}Nb_{0,11}Ni_{0,01}$ | 0,26 | 390 | 30 | 66 | 9 |
| B1 | $Mo_1V_{0,29}Te_{0,14}Nb_{0,13}Ni_{0,007}$ | 0,71 | 390 | 80 | 66 | 2 |
| VB2 | $Mo_1V_{0,33}Te_{0,19}Nb_{0,11}Pd_{0,01}$ | 0,25 | 390 | 80 | 62 | 1 |
| B2 | $Mo_1V_{0,28}Te_{0,13}Nb_{0,13}Pd_{0,001}$ | 0,73 | 420 | 77 | 59 | 1 |
| VB3 | $Mo_1V_{0,33}Te_{0,22}Nb_{0,11}Pd_{0,04}$ | 0,35 | 440 | 75 | 42 | 1 |
| B3 | $Mo_1V_{0,29}Te_{0,13}Nb_{0,13}Pd_{0,001}$ | 0,74 | 385 | 77 | 60 | 1 |
| VB4 | $Mo_1V_{0,33}Te_{0,19}Nb_{0,11}Co_{0,005}$ | 0,24 | 440 | 79 | 44 | 1 |
| B4 | $Mo_1V_{0,29}Te_{0,13}Nb_{0,13}Co_{0,004}$ | 0,73 | 390 | 76 | 62 | 2 |
| VB5 | $Mo_1V_{0,33}Te_{0,19}Nb_{0,11}Cu_{0,01}$ | 0,27 | 420 | 59 | 56 | 3 |
| B5 | $Mo_1V_{0,28}Te_{0,13}Nb_{0,13}Cu_{0,003}$ | 0,74 | 420 | 73 | 62 | 2 |
| VB6 | $Mo_1V_{0,33}Te_{0,19}Nb_{0,11}Bi_{0,004}$ | 0,18 | 400 | 83 | 54 | 1 |
| B6 | $Mo_1V_{0,28}Te_{0,15}Nb_{0,14}Bi_{0,005}$ | 0,70 | 410 | 77 | 62 | 1 |
| VB7 | $Mo_1V_{0,34}Te_{0,18}Nb_{0,11}Pb_{0,004}$ | 0,30 | 440 | 78 | 43 | 1 |
| B7 | $Mo_1V_{0,28}Te_{0,13}Nb_{0,13}Pb_{0,001}$ | 0,67 | 420 | 78 | 58 | 2 |
| VB8 | $Mo_1V_{0,33}Te_{0,16}Nb_{0,11}$ | 0,26 | 420 | 68 | 55 | 2 |
| VB9 | $Mo_1V_{0,29}Te_{0,13}Nb_{0,13}$ | 0,68 | 410 | 80 | 56 | 2 |

**Patentansprüche**

1. Multimetalloxidmasse der allgemeinen Stöchiometrie I

$$Mo_1V_aM^1_bM^2_cM^3_dO_n \qquad (I),$$

mit

M$^1$ = wenigstens eines der Elemente aus der Gruppe umfassend Te und Sb;
M$^2$ = wenigstens eines der Elemente aus der Gruppe umfassend Nb, Ti, W, Ta und Ce;
M$^3$ = wenigstens eines der Elemente aus der Gruppe umfassend Pb, Ni, Co, Bi, Pd, Ag, Pt, Cu, Au, Ga, Zn, Sn, In, Re, Ir, Sm, Sc, Y, Pr, Nd und Tb;
a = 0,01 bis 1,
b = > 0 bis 1,
c = > 0 bis 1,
d = > 0 bis 0,5 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2Θ) 22,2 ± 0,5° (h), 27,3 ± 0,5° (i) und 28,2 ± 0,5° (k) liegen, wobei

- der Beugungsreflex h innerhalb des Röntgendiffraktogramms der intensitätsstärkste ist, sowie eine Halbwertsbreite von höchstens 0,5° aufweist,
- die Intensität Pi des Beugungsreflexes i und die Intensität $P_k$ des Beugungsreflexes k die Beziehung $0,65 \leq R \leq 0,85$ erfüllen, in der R das durch die Formel

$$R = P_i \; / \; (P_i + P_k)$$

- die Halbwertsbreite des Beugungsreflexes i und des Beugungsreflexes k jeweils $\leq 1°$ beträgt,

**dadurch gekennzeichnet, daß** die wenigstens eine Multimetalloxidmasse (I) eine solche ist, deren Röntgendiffraktogramm keinen Beugungsreflex mit der Scheitelpunktlage $2\Theta = 50,0 \pm 0,3°$ aufweist.

2. Multimetalloxidmasse nach Anspruch 1, **dadurch gekennzeichnet, dass** $0,67 \leq R \leq 0,75$.

3. Multimetalloxidmasse nach Anspruch 1, **dadurch gekennzeichnet, dass** $0,69 \leq R \leq 0,75$.

4. Multimetalloxidmasse nach Anspruch 1, **dadurch gekennzeichnet, dass** $0,71 \leq R \leq 0,74$.

5. Multimetalloxidmasse nach Anspruch 1, **dadurch gekennzeichnet, dass** $R = 0,72. \leq$

6. Multimetalloxidmasse nach einem der Ansprüche 1 bis 5, **dadurch gekennzeichnet, dass** ihre spezifische Oberfläche 11 bis 40 m$^2$/g beträgt.

7. Multimetalloxidmasse nach einem der Ansprüche 1 bis 6, **dadurch gekennzeichnet, dass** ihr Röntgendiffraktogramm noch weitere Beugungsreflexe mit ihrer Scheitelpunktlage bei den nachfolgenden Beugungswinkeln-$2\Theta$ aufweist:

$9,0 \pm 0,4°$ (l),
$6,7 \pm 0,4°$ (o), und
$7,9 \pm 0,4°$ (p).

8. Multimetalloxidmasse nach Anspruch 7, **dadurch gekennzeichnet, daß** ihr Röntgendiffraktogramm noch weitere Beugungsreflexe mit ihrer Scheitelpunktlage bei den nachfolgenden Beugungswinkeln-20 aufweist:

$45,2 \pm 0,4°$ (q),
$29,2 \pm 0,4°$ (m), und
$35,4 \pm 0,4°$ (n).

9. Multimetalloxidmasse nach Anspruch 8, **dadurch gekennzeichnet, daß** die Intensität der Beugungsreflexe h, i, l, m, n, o, p, q in der gleichen Intensitätsskala die nachfolgenden Intensitäten aufweisen:

h = 100,
i = 5 bis 95,
l = 1 bis 30,
m = 1 bis 40,
n = 1 bis 40,
o = 1 bis 30,
p = 1 bis 30, und
q = 5 bis 60.

10. Multimetalloxidmassen nach einem der Ansprüche 1 bis 9, **dadurch gekennzeichnet, dass** a = 0,05 bis 0,6.

11. Multimetalloxidmasse nach einem der Ansprüche 1 bis 10, **dadurch gekennzeichnet, dass** b = 0,01 bis 1.

12. Multimetalloxidmasse nach einem der Ansprüche 1 bis 11, **dadurch gekennzeichnet, dass** c = 0,01 bis 1.

**13.** Multimetalloxidmasse nach einem der Ansprüche 1 bis 12, **dadurch gekennzeichnet, dass** d = 0,0005 bis 0,5.

**14.** Multimetalloxidmasse nach einem der Ansprüche 1 bis 13, **dadurch gekennzeichnet, dass**

a = 0,1 bis 0,6;
b = 0,1 bis 0,5;
c = 0,1 bis 0,5 und
d = 0,001 bis 0,5.

**15.** Multimetalloxidmasse nach einem der Ansprüche 1 bis 14, **dadurch gekennzeichnet, dass** $M^2$ zu wenigstens 50 mol-% seiner Gesamtmenge Nb ist.

**16.** Multimetalloxidmasse nach einem der Ansprüche 1 bis 14, **dadurch gekennzeichnet, dass** $M^2$ zu wenigstens 75 mol-% seiner Gesamtmenge Nb ist.

**17.** Multimetalloxidmasse nach einem der Ansprüche 1 bis 14, **dadurch gekennzeichnet, dass** $M^2$ ausschließlich Nb ist.

**18.** Multimetalloxidmasse nach einem der Ansprüche 1 bis 17, **dadurch gekennzeichnet, dass** $M^3$ wenigstens ein Element aus der Gruppe umfassend Ni, Co, Bi, Pd, Ag, Au, Pb und Ga ist.

**19.** Multimetalloxidmasse nach einem der Ansprüche 1 bis 17, **dadurch gekennzeichnet, dass** $M^3$ wenigstens ein Element aus der Gruppe umfassend Ni, Co, Pd und Bi ist.

**20.** Multimetalloxidmasse nach einem der Ansprüche 1 bis 17, **dadurch gekennzeichnet, dass** $M^1$ = Te, $M^2$ = Nb und $M^3$ = wenigstens ein Element aus der Gruppe umfassend Ni, Co und Pd.

**21.** Multimetalloxidmasse, die wenigstens eine Multimetalloxidmasse gemäß einem der Ansprüche 1 bis 20 enthält und deren Röntgendiffraktogramm keinen Beugungsreflex mit der Scheitelpunktlage 2Θ = 50,0 ± 0,3° aufweist.

**22.** Multimetalloxidmasse nach Anspruch 21, in der die Multimetalloxidmasse (I) in mit wenigstens einem feinteiligen Material aus der Gruppe umfassend Siliciumdioxid, Titandioxid, Aluminiumoxid, Zirkonoxid und Nioboxid verdünnter Form vorliegt.

**23.** Multimetalloxidmasse, die zu ≥ 80 Gew.-% wenigstens eine Multimetalloxidmasse gemäß einem der Ansprüche 1 bis 20 enthält und deren Röntgendiffraktogramm einen Beugungsreflex mit der Scheitelpunktlage 2Θ = 50,0 ± 0,3° aufweist.

**24.** Verfahren der heterogen katalysierten partiellen Gasphasenoxidation wenigstens eines gesättigten oder ungesättigten Kohlenwasserstoffs, **dadurch gekennzeichnet, dass** als katalytische Aktivmasse wenigsten eine Multimetalloxidmasse gemäß einem der Ansprüche 1 bis 23 verwendet wird.

**25.** Verfahren nach Anspruch 24, **dadurch gekennzeichnet, daß** der Kohlenwasserstoff Propan, Propen oder ein Gemisch aus Propan und Propen ist.

**26.** Verfahren der heterogen katalysierten partiellen Gasphasenammoxidation wenigstens eines gesättigten oder ungesättigten Kohlenwasserstoffs, **dadurch gekennzeichnet, dass** als katalytische Aktivmasse wenigstens eine Multimetalloxidmasse gemäß einem der Ansprüche 1 bis 23 verwendet wird.

**27.** Verfahren nach Anspruch 26, **dadurch gekennzeichnet, daß** der Kohlenwasserstoff Propan, Propen oder ein Gemisch aus Propan und Propen ist.

**28.** Verwendung wenigstens einer Multimetalloxidmasse gemäß einem der Ansprüche 1 bis 23 als Katalysator für eine heterogen katalysierte partielle Oxidation und/oder Ammoxidation wenigstens eines gesättigten und/oder ungesättigten Kohlenwasserstoffs.

**29.** Verfahren zur Herstellung eine Multimetalloxidmasse gemäß einem der Ansprüche 1 bis 20, **dadurch gekennzeichnet, dass** man aus Quellen ihrer elementaren Konstituenten ein inniges Trockengemisch erzeugt, dieses bei Temperaturen von 350 bis 700°C calciniert und das dabei resultierende Produkt mit einer wäßrigen Lösung einer organischen und/oder anorganischen Säure wäscht.

**Claims**

1.  A multimetal oxide material of the stoichiometry I

$$Mo_1V_aM^1_bM^2_cM^3_dO_n \qquad (I),$$

    where

    $M^1$ is at least one of the elements from the group consisting of Te and Sb;
    $M^2$ is at least one of the elements from the group consisting of Nb, Ti, W, Ta and Ce;
    $M^3$ is at least one of the elements from the group consisting of Pb, Ni, Co, Bi, Pd, Ag, Pt, Cu, Au, Ga, Zn, Sn, In, Re, Ir, Sm, Sc, Y, Pr, Nd and Tb;
    a is from 0.01 to 1,
    b is from > 0 to 1,
    c is from > 0 to 1,
    d is from > 0 to 0.5 and
    n is a number which is determined by the valency and frequency of the elements other than oxygen in (I),

    whose X-ray diffraction pattern has reflections h, i and k whose peaks are at the diffraction angles (2θ) 22.2 $\pm$ 0.5° (h), 27.3 $\pm$ 0.5° (i) and 28.2 $\pm$ 0.5° (k),

    -   the reflection h being the one with the strongest intensity within the X-ray diffraction pattern and having a full width at half height (FWHH) of not more than 0.5°,
    -   the intensity $P_i$ of the reflection i and the intensity $P_k$ of the reflection k fulfilling the relationship $0.65 \leq R \leq 0.85$, where R is the intensity ratio defined by the formula

    $$R \; = \; P_i \; / \; (P_i \; + \; P_k)$$

    and
    -   the FWHH of the reflection i and of the reflection k is in each case $\leq 1°$,

    wherein the at least one multimetal oxide material (I) is one whose X-ray diffraction pattern has no reflection with the peak position $2\theta = 50.0 \pm 0.3°$.

2.  The multimetal oxide material according to claim 1, wherein $0.67 \leq R \leq 0.75$.

3.  The multimetal oxide material according to claim 1, wherein $0.69 \leq R \leq 0.75$.

4.  The multimetal oxide material according to claim 1, wherein $0.71 \leq R \leq 0.74$.

5.  The multimetal oxide material according to claim 1, wherein $R = 0.72$.

6.  The multimetal oxide material according to any of claims 1 to 5, wherein its specific surface area is from 11 to 40 $m^2/g$.

7.  The multimetal oxide material according to any of claims 1 to 6, wherein its X-ray diffraction pattern also has further reflections with their peak positions at the following diffraction angles 2θ:

    9.0 $\pm$ 0.4° (1),
    6.7 $\pm$ 0.4° (o) and
    7.9 $\pm$ 0.4° (p).

8.  The multimetal oxide material according to claim 7, wherein its X-ray diffraction pattern also has further reflections with their peak positions at the following diffraction angles 2θ:

45.2 $\pm$ 0.4° (q)
29.2 2 $\pm$ 0.4° (m) and
35.4 $\pm$ 0.4° (n).

9. The multimetal oxide material according to claim 8, wherein, on the same intensity scale, the reflections h, i, l, m, n, o, p and q have the following intensities:

h = 100,
i = from 5 to 95,
1 = from 1 to 30,
m = from 1 to 40,
n = from 1 to 40,
o = from 1 to 30,
p = from 1 to 30 and
q = from 5 to 60.

10. The multimetal oxide material according to any of claims 1 to 9, wherein a is from 0.05 to 0.6.

11. The multimetal oxide material according to any of claims 1 to 10, wherein b is from 0.01 to 1.

12. The multimetal oxide material according to any of claims 1 to 11, wherein c is from 0.01 to 1.

13. The multimetal oxide material according to any of claims 1 to 12, wherein d is from 0.0005 to 0.5.

14. The multimetal oxide material according to any of claims 1 to 13, wherein

a = from 0.1 to 0.6;
b = from 0.1 to 0.5;
c = from 0.1 to 0.5 and
d = from 0.001 to 0.5.

15. The multimetal oxide material according to any of claims 1 to 14, wherein $M^2$ comprises at least 50 mol%, based on its total weight, of Nb.

16. The multimetal oxide material according to any of claims 1 to 14, wherein $M^2$ comprises at least 75 mol%, based on its total weight, of Nb.

17. The multimetal oxide material according to any of claims 1 to 14, wherein $M^2$ is exclusively Nb.

18. The multimetal oxide material according to any of claims 1 to 17, wherein $M^3$ is at least one element from the group consisting of Ni, Co, Bi, Pd, Ag, Au, Pb and Ga.

19. The multimetal oxide material according to any of claims 1 to 17, wherein $M^3$ is at least one element from the group consisting of Ni, Co, Pd and Bi.

20. The multimetal oxide material according to any of claims 1 to 17, wherein $M^1$ is Te, $M^2$ is Nb and $M^3$ is at least one element from the group consisting of Ni, Co and Pd.

21. A multimetal oxide material which comprises at least one multimetal oxide material according to any of claims 1 to 20 and whose X-ray diffraction pattern has no reflection with the peak position 2θ = 50.0 $\pm$ 0.3°.

22. The multimetal oxide material according to claim 21, in which the multimetal oxide material (I) is present in a form diluted with at least one finely divided material from the group consisting of silica, titanium dioxide, alumina, zirconium oxide and niobium oxide.

23. A multimetal oxide material which comprises $\geq$ 80% by weight of at least one multimetal oxide material according to any of claims 1 to 20 and whose X-ray diffraction pattern has a reflection with the peak position 2θ = 50.0 $\pm$ 0.3°.

24. A process for the heterogeneously catalyzed partial gas-phase oxidation of at least one saturated or unsaturated hydrocarbon, wherein the catalytically active material used is at least one multimetal oxide material according to any of claims 1 to 23.

25. The process according to claim 24, wherein the hydrocarbon is propane, propene or a mixture of propane and propene.

26. A process for the heterogeneously catalyzed partial gas-phase ammoxidation of at least one saturated or unsaturated hydrocarbon, wherein the catalytically active material used is at least one multimetal oxide material according to any of claims 1 to 23.

27. The process according to claim 26, wherein the hydrocarbon is propane, propene or a mixture of propane and propene.

28. The use of at least one multimetal oxide material according to any of claims 1 to 23 as a catalyst for a heterogeneously catalyzed partial oxidation and/or ammoxidation of at least one saturated and/or unsaturated hydrocarbon.

29. A process for the preparation of a multimetal oxide material according to any of claims 1 to 20, wherein a thorough dry mixture is produced from sources of the elemental constituents of multimetal oxide material, said mixture is calcined at from 350 to 700°C and the resulting product is washed with an aqueous solution of an organic and/or inorganic acid.

**Revendications**

1. Masse d'oxyde multimétallique de stoechiométrie générale 1

$$Mo_1V_aM^1{}_bM^2{}_cM^3{}_dO_n \qquad (I),$$

où
$M^1$ = au moins un des éléments du groupe incluant Te et Sb ;
$M^2$ = au moins un des éléments du groupe incluant Nb, Ti, W, Ta et Ce ;
$M^3$ = au moins un des éléments du groupe incluant Pb, Ni, Co, Bi, Pd, Ag, Pt, Cu, Au, Ga, Zn, Sn, In, Re, Ir, Sm, Sc, Y, Pr, Nd et Tb ;

a = 0,01 à 1,
b => 0 à 1,
c => 0 à 1,
d=> 0 à 0,5 et
n= un nombre qui est déterminé par la valeur et la fréquence des éléments différents de l'oxygène dans (I),

dont le diffractogramme radiographique présente des réflexes de flexion h, i et k dont les points sommitaux se situent aux angles de flexion (2θ) 22,2 2 $\pm$ 0,5° (h), 27,3 $\pm$ 0,5° (i) et 28,2 $\pm$ 0,5° (k), sachant que

- le réflexe de flexion h se situe dans le cadre du diffractogramme radiographique de la plus forte intensité et présente une largeur de raies spectrales de 0,5° au maximum,
- l'intensité $P_i$ du réflexe de flexion i et l'intensité $P_k$ du réflexe de flexion k répondent à l'équation $0,65 \leq R \leq 0,85$, dans laquelle R est le rapport d'intensité défini par la formule

$$R = P_i / (P_i + P_k)$$

et
- la largeur de raies spectrales du réflexe de flexion i et du réflexe de flexion k est respectivement $\leq 1°$,

**caractérisée en ce que** l'au moins une masse d'oxyde multimétallique (I) est une masse dont le diffractogramme radiographique ne présente pas de réflexe de flexion à la position de point sommital 2θ = 50,0 ± 0,3°.

**2.** Masse d'oxyde multimétallique selon la revendication 1, **caractérisée en ce que** 0,67 ≤ R ≤ 0,75.

**3.** Masse d'oxyde multimétallique selon la revendication 1, **caractérisée en ce que** 0,69 ≤ R ≤ 0,75.

**4.** Masse d'oxyde multimétallique selon la revendication 1, **caractérisée en ce que** 0,71 ≤ R ≤ 0,74.

**5.** Masse d'oxyde multimétallique selon la revendication 1, **caractérisée en ce que** R = 0,72.

**6.** Masse d'oxyde multimétallique selon une des revendications 1 à 5, **caractérisée en ce que** sa surface spécifique 11 peut atteindre 40 m$^2$/g.

**7.** Masse d'oxyde multimétallique selon une des revendications 1 à 6, **caractérisée en ce que** son diffractogramme radiographique présente encore d'autres réflexes de flexion à sa position de point sommital aux angles de flexion 2θ suivants :

9,0 ± 0,4° (1),
6,7 ± 0,4° (o), et
7,9 ± 0,4° (p).

**8.** Masse d'oxyde multimétallique selon la revendication 7, **caractérisée en ce que** son diffractogramme radiographique présente encore d'autres réflexes de flexion à sa position de point sommital aux angles de flexion 2θ suivants :

45,2 ± 0,4° (q),
29,2 ± 0,4° (m), et
35,4 ± 0,4° (n).

**9.** Masse d'oxyde multimétallique selon la revendication 8, **caractérisée en ce que** l'intensité des réflexes de flexion h, i, 1, m, n, o, p, q dans la même échelle d'intensité présente les valeurs suivantes :

h = 100,
i = 5 à 95,
l = 1 à 30,
m = 1 à 40,
n = 1 à 40,
o = 1 à 30,
p = 1 à 30, et
q = 5 à 60.

**10.** Masse d'oxyde multimétallique selon une des revendications 1 à 9, **caractérisée en ce que** a = 0,05 à 0,6.

**11.** Masse d'oxyde multimétallique selon une des revendications 1 à 10, **caractérisée en ce que** b = 0,01 à 1.

**12.** Masse d'oxyde multimétallique selon une des revendications 1 à 11, **caractérisée en ce que** c = 0,01 à 1.

**13.** Masse d'oxyde multimétallique selon une des revendications 1 à 12, **caractérisée en ce que** d = 0,0005 à 0,5.

**14.** Masse d'oxyde multimétallique selon une des revendications 1 à 13, **caractérisée en ce que**

a = 0,1 à 0,6;
b = 0,1 à 0,5;
c = 0,1 à 0,5, et
d = 0,001 à 0,5.

**15.** Masse d'oxyde multimétallique selon une des revendications 1 à 14, **caractérisée en ce que** M$^2$ est formé de Nb pour au moins 50 % en moles de sa quantité totale.

**16.** Masse d'oxyde multimétallique selon une des revendications 1 à 14, **caractérisée en ce que** $M^2$ est formé de Nb pour au moins 75 % en moles de sa quantité totale.

**17.** Masse d'oxyde multimétallique selon une des revendications 1 à 14, **caractérisée en ce que** $M^2$ est exclusivement formé de Nb.

**18.** Masse d'oxyde multimétallique selon une des revendications 1 à 17, **caractérisée en ce que** $M^3$ est au moins un élément du groupe composé de Ni, Co, Bi, Pd, Ag, Au, Pb et Ga.

**19.** Masse d'oxyde multimétallique selon une des revendications 1 à 17, **caractérisée en ce que** $M^3$ est au moins un élément du groupe composé de Ni, Co, Pd et Bi.

**20.** Masse d'oxyde multimétallique selon une des revendications 1 à 17, **caractérisée en ce que** $M^1$ = Te, $M^2$ = Nb et $M^3$ = au moins un élément du groupe composé de Ni, Co et Pd.

**21.** Masse d'oxyde multimétallique contenant au moins une masse d'oxyde multimétallique selon une des revendications 1 à 20 et dont le diffractogramme radiographique ne présente pas de réflexe de flexion à la position de point sommital $2\theta = 50{,}0 \pm 0{,}3°$.

**22.** Masse d'oxyde multimétallique selon la revendication 21, dans laquelle la masse d'oxyde multimétallique (I) se présente sous forme diluée avec au moins un matériau à particules fines du groupe comprenant le dioxyde de silicium, le dioxyde de titane, l'oxyde d'aluminium, l'oxyde de zirconium et l'oxyde de niobium.

**23.** Masse d'oxyde multimétallique qui contient à $\geq$ 80 % en poids au moins une masse d'oxyde multimétallique selon une des revendications 1 à 20 et dont le diffractogramme radiographique présente un réflexe de flexion à la position de point sommital $2\theta = 50{,}0 \pm 0{,}3°$.

**24.** Procédé d'oxydation en phase gazeuse partielle à catalyse hétérogène d'au moins un hydrocarbure saturé ou insaturé, **caractérisé en ce qu'**on utilise comme masse active catalytique au moins une masse d'oxyde multimétallique selon une des revendications 1 à 23.

**25.** Procédé selon la revendication 24, **caractérisé en ce que** l'hydrocarbure est du propane, du propène ou un mélange de propane et propène.

**26.** Procédé d'ammoxydation en phase gazeuse partielle à catalyse hétérogène d'au moins un hydrocarbure saturé ou insaturé, **caractérisé en ce qu'**on utilise comme masse active catalytique au moins une masse d'oxyde multimétallique selon une des revendications 1 à 23.

**27.** Procédé selon la revendication 26, **caractérisé en ce que** l'hydrocarbure est du propane, du propène ou un mélange de propane et propène.

**28.** Utilisation d'au moins une masse d'oxyde multimétallique selon une des revendications 1 à 23 comme catalyseur pour une oxydation partielle à catalyse hétérogène et/ou ammoxydation d'au moins un hydrocarbure saturé et/ou insaturé.

**29.** Procédé de fabrication d'une masse d'oxyde multimétallique selon une des revendications 1 à 20, **caractérisé en ce qu'**on prépare à partir de sources de ses constituants élémentaires un mélange intime sec, qu'on le calcine à des températures de 350 à 700° C et qu'on lave le produit en résultant avec une solution aqueuse d'un acide organique et/ou anorganique.

# FIG.1

# FIG.2

2-Theta [°]

# FIG.3

2-Theta [°]

# FIG.4

2-Theta [°]

# FIG.5

2-Theta [°]

# FIG.6

2-Theta [°]

# FIG.7

2-Theta [°]

# FIG.8

2-Theta [°]

# FIG.9

2-Theta [°]

# FIG.10

2-Theta [°]

# FIG.11

2-Theta [°]

EP 1 558 569 B1

# FIG.12

37

# FIG.13

2-Theta [°]

# FIG.14

2-Theta [°]

# FIG.15

2-Theta [°]

# FIG.16

2-Theta [°]

# FIG.17

2-Theta [°]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 318295 A
- EP 512846 A
- EP 529853 A
- EP 603836 A
- EP 608838 A
- EP 767164 A
- EP 895809 A
- EP 962253 A
- DE 19835247 A
- EP 1090684 A
- WO 0206199 A
- EP 1192987 A
- EP 1192986 A
- EP 1192983 A
- EP 1192982 A
- JP 11169716 A
- EP 895089 A
- JP 7232071 A
- WO 0029106 A
- WO 0029105 A
- WO 0038833 A
- WO 0069802 A
- DE 10118814 A
- EP 0204073 W
- JP 8057319 A
- DE 10122027 A
- DE 10051419 A
- DE 10046672 A
- EP 1080784 A
- EP 1123738 A
- EP 1192988 A
- DE 10145958 A
- JP 7315842 A
- DE 10029338 A
- JP 2000143244 A
- DE 2909671 A
- DE 10101695 A
- WO 9822421 A
- DE 10246119 A
- JP 10036311 A
- DE 19924532 A
- JP 7053448 A
- DE 2351151 A
- JP 6166668 A
- JP 6211767 A
- US 4250346 A
- EP 261264 B

**In der Beschreibung aufgeführte Nicht-Patentliteratur**

- Ammoxidation of propane over Mo-V-Nb-Te mixed oxide catalysts. Spillover and Migration of Surface on Catalysts. Elsevier Science B.V, 1997, 473 ff
- *Polyhedron,* 1987, vol. 6 (2), 213-218
- **vom Anderson.** *Typ bildet Kinetics and Catalysis,* 1999, vol. 40 (3), 401-404